(19) Europäisches Patentamt / European Patent Office / Office européen des brevets

(11) **EP 4 406 529 A1**

(12) **DEMANDE DE BREVET EUROPEEN**

(43) Date de publication:
**31.07.2024 Bulletin 2024/31**

(21) Numéro de dépôt: **24154088.9**

(22) Date de dépôt: **26.01.2024**

(51) Classification Internationale des Brevets (IPC):
**A61K 8/34** *(2006.01)* **A61K 8/97** *(2017.01)*
**A61Q 7/00** *(2006.01)* **A61Q 19/08** *(2006.01)*

(52) Classification Coopérative des Brevets (CPC):
**A61K 8/97; A61K 8/347; A61Q 7/00; A61Q 19/08**

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**GE KH MA MD TN**

(30) Priorité: **26.01.2023 FR 2300716**

(71) Demandeur: **Mondin, Jean-Daniel**
**06200 Nice (FR)**

(72) Inventeur: **MONDIN, Jean-Daniel**
**06200 NICE (FR)**

(74) Mandataire: **Novagraaf Technologies**
**Bâtiment O2**
**2, rue Sarah Bernhardt**
**CS90017**
**92665 Asnières-sur-Seine Cedex (FR)**

(54) **COMPOSITION COSMETIQUE REGENERANTE**

(57) La présente invention concerne une composition cosmétique destinée à régénérer l'épiderme et le follicule pileux pour lutter contre le vieillissement de la peau et ralentir la chute et la dépigmentation des cheveux, comprenant de l'éther vanillyl-butylique et un extrait de graines de *Voandzeia subterranea*, et optionnellement un milieu nutricellulaire pour renforcer l'effet des deux substances actives.

[Fig. 7]

Evolution of the mean perfusion on the scalp 30 minutes after the application of the HAIR SUMMUM SERUM NEW LIFE VR021195.A05 + DEVICE HAIR SUMMUM

EP 4 406 529 A1

**Description**

**Domaine technique**

La présente invention concerne une composition cosmétique non thérapeutique comprenant de l'éther vanillyl-butylique et un extrait de graines de *Voandzeia subterranea,* et optionnellement un milieu nutricellulaire pour renforcer l'effet des deux substances actives.

La composition cosmétique non thérapeutique de la présente invention à effet vasodilatateur et de ré-innervation cutanée est destinée à lutter contre le vieillissement de la peau, en particulier en réduisant la taille des rides présentes sur la peau à des fins non thérapeutiques. Elle a également une action au niveau du bulbe pileux pour ralentir la chute et la dépigmentation des cheveux.

**Etat de la technique**

La peau possède trois couches biologiques différentes :

A/ L'épiderme est constitué de cellules constamment renouvelées par la couche basale, à son tour formée de cellules reproductrices.
B/ Le derme est constitué d'un gel de mucopolysaccharides et d'eau dans lequel sont baignées des fibres élastiques.
C/ L'hypoderme est la partie la plus profonde de la peau. Elle est intensément vascularisée, elle contient localement des cellules adipeuses.

Comme la peau, le cuir chevelu est composé de trois couches superposées, qui sont l'épiderme, le derme et l'hypoderme. Il s'en distingue du fait qu'il contient un nombre important de follicules pileux produisant les cheveux et par la présence d'un plus grand nombre de glandes sébacées que pour d'autres parties du corps. Le cuir chevelu est également richement vascularisé et comprend de nombreuses terminaisons nerveuses. La vascularisation du follicule pileux est essentielle au bon développement et à la croissance du cheveu, plus particulièrement au moment de la phase anagène. Elle participe aux processus impliqués dans la régulation du cycle du cheveu, au cours duquel des facteurs de croissance, des cytokines et d'autres molécules sont impliquées. Le VEGF (Vascular-Endothelial Growth Factor) ou facteur de vascularisation agit directement sur les papilles dermiques et stimule la formation du réseau vasculaire situé à proximité du follicule pileux (Kozlowska et al, 1998, Yano et al, 2001) [1, 2] ce qui permet de répondre aux besoins nutritionnels du follicule pileux particulièrement au cours de la phase anagène. C'est au sein du follicule pileux qu'interviennent les mécanismes à l'origine de la pigmentation du cheveu. Ainsi, la large variété de couleurs de cheveux est le résultat d'un savant mélange entre deux pigments nommés l'eumélanine (noir-brun) et phéomélanine (brun-rouge-jaune). Ces deux types de mélanine sont synthétisés par les mélanocytes, cellules spécialisées dans la mélanogenèse, à partir d'un acide aminé précurseur qui est la tyrosine ; la tyrosine étant également synthétisée à partir de la phénylalanine. Localisés au niveau de la papille dermique du follicule pileux, les mélanocytes transfèrent les mélanosomes aux kératinocytes pré-corticaux voisins qui se différencient et s'assemblent pour former la tige pilaire pigmentée en croissance (Fernandez-Flores et al, 2019) [3].

La peau (y compris le cuir chevelu) est innervée par deux types de nerfs : les nerfs sensoriels et les nerfs autonomes. Les nerfs autonomes dépendent du système nerveux autonome, c'est à dire la partie du cerveau en charge de la régulation des fonctions automatiques de l'organisme telles que la respiration, la digestion, les muscles cardiaques ou encore le maintien de l'homéostasie cutanée.

Le maintien de l'homéostasie cutanée passe entre autres par le renouvellement et la maturation des kératinocytes, et dans l'organisation épidermique. Il a été démontré que l'organisation de l'épiderme est directement reliée à son degré d'innervation : 30 à 40% de l'épaisseur de l'épiderme est contrôlée par la libération de neurotransmetteurs dans la peau (Hsieh ST et Lin WM., 1999) [4].

Avec l'âge, l'innervation de la peau est réduite et l'épiderme s'amincie. Heureusement, ce phénomène est réversible et l'épiderme peut retrouver son épaisseur normale si l'innervation est renouvelée.

La principale conséquence visible du vieillissement cutané est une accentuation de la rugosité de la peau, des rides et des ridules, et une perte d'éclat. La ride correspond à un affaissement symétrique de l'extérieur et de l'intérieur de l'épiderme, et le maintien du sillon de la ride résulte de la solidification des fibres élastiques du derme. L'hypoderme a quant à lui peu d'importance dans l'apparition des rides. Actuellement il y a 3 solutions pour diminuer l'apparence des rides :

A/ La chirurgie esthétique avec la technique du lifting, le chirurgien resserre la peau du visage. L'effet de tension horizontale lisse les rides et diminue leur apparence.
B/ Les injections de substances biologiques de remplissage. Le processus consiste à injecter sous l'épiderme et plus particulièrement sous la ride des substances gélifiées. L'apport de substances permet de remplir l'intérieur de la ride et de diminuer ainsi son aspect extérieur.

C/ L'utilisation de crèmes antirides. Ce processus consiste à appliquer chaque jour sur le visage des émulsions contenant des principes actifs dont l'efficacité a été démontrée pour leurs actions anti-âge ou antirides. Ces crèmes sont appliquées pendant environ 4 à 6 semaines selon leur temps d'utilisation.

Les solutions existantes ne sont malheureusement pas applicables à tous les types de peau et certaines sont très coûteuses et intrusives.

Concernant plus particulièrement le cuir chevelu (*i.e.* partie de peau du crâne), avec l'âge, les lésions ou pathologies dermatologiques, des cheveux blancs apparaissent et la chute des cheveux normalement ancrés dans le derme peut s'accentuer pouvant aller jusqu'à l'alopécie. Plusieurs facteurs sont en cause : chez l'homme : les hormones androgènes et la génétique ; chez la femme : le stress, les périodes post-partum, les bouleversements hormonaux (cycles menstruels conséquences de modifications brutales du poids, de la ménopause, des contraceptifs et des traitements hormonaux de substitution), les carences en micronutriments (*e.g.* fer), une alimentation dénaturée et une médicalisation en constante augmentation.

Il existe donc un besoin pour une solution alternative permettant de lutter contre le vieillissement cutané, y compris du cuir chevelu (*i.e.* réduire les rides sur la peau, limiter la chute des cheveux et favoriser leur repousse, retarder l'apparition des cheveux blancs), sans qu'il soit nécessaire d'introduire un objet dans la peau et qui soit plus efficace que les compositions actuelles.

## Description de l'invention

Les inventeurs ont mis au point une nouvelle composition cosmétique à effet vasodilatateur et de ré-innervation cutanée destinée à lutter contre les signes du vieillissement de la peau, y compris du cuir chevelu (ralentissement de la régénération cellulaire (kératinocytes), ralentissement du métabolisme cellulaire (kératinocytes, mélanocytes), affinement de l'épiderme et relâchement cutané, etc...). Ladite composition comprend de l'éther vanillyl-butylique et un extrait de graines de *Voandzeia subterranea* (permettant d'activer la microcirculation et l'innervation cutanée, respectivement), et optionnellement un milieu nutricellulaire pour renforcer l'effet des deux substances actives.

L'éther vanillyl-butylique génère une sensation de chaleur, et favorise la vasodilatation. Cet actif chauffant atténue les conséquences du relâchement cutané (*i.e.* stagnation des fluides sous cutanés) par son effet vasodilatateur. Son action repose sur un groupement spécifique de la molécule, qui par sa structure est capable d'interagir avec les récepteurs vanilloïdes VR-1 présents sur la membrane des cellules. Lorsqu'il se fixe sur la membrane des cellules, une cascade de signalisation est amorcée à l'intérieur de la cellule et aboutit à un signal neurologique de chaleur et de vasodilatation. Grâce à son effet vasodilatateur et à la génération d'une sensation de chaleur douce et continue, il joue sur l'accroissement de la microcirculation pour favoriser le métabolisme sous-cutané. L'afflux sanguin est augmenté localement, pour une peau mieux nourrie et oxygénée. De plus, la chaleur dilate les pores de la peau et facilite ainsi la pénétration des actifs. L'éther vanillyl-butylique a donc une action au niveau cutané mais également au niveau du cuir chevelu car l'augmentation de la microcirculation entraîne une amélioration de l'apport en oxygène et en nutriments, comme les acides aminés (tyrosine, phénylalanine...) et les facteurs de croissance (VEGF , IGF-1 et bFGF), au niveau du follicule pileux pour une stimulation du métabolisme cellulaire, une stimulation de la croissance du cheveu ; une amélioration de la différenciation des cellules du bulbe pileux et donc une amélioration de la pigmentation du cheveu.. La fibre capillaire, en phase anagène, est donc fortifiée, sa qualité structurale est améliorée et son diamètre est visiblement augmenté.

L'extrait de graines de *Voandzeia subterranea* est un actif anti-âge qui favorise l'innervation de l'épiderme et aide à lutter contre l'affinement de l'épiderme. Cet actif contribue à rajeunir l'apparence de la peau et à la maintenir en bon état en relançant l'innervation cutanée. Grâce à une meilleure innervation, le mécanisme d'affinement de l'épiderme lié au vieillissement cutané est inversé, la prolifération des kératinocytes est stimulée et ainsi les signes de l'âge sont atténués. D'autre part, il contribue à la protection des cellules cutanées (maintient une bonne viabilité cellulaire) et renforce la structure de la peau en stimulant la cohésion cellulaire grâce aux desmosomes (jonctions entre les cellules épidermiques).

Le milieu nutricellulaire contribue à relancer la régénération cellulaire (kératinocytes), à activer le métabolisme cellulaire (kératinocytes, mélanocytes) et assure un apport en tyrosine et phénylalanine (précurseurs de la mélanine). Ce milieu est composé d'au moins une vitamine ou composé assimilé à une vitamine, d'au moins deux acides aminés (dont au moins la phénylalanine et la tyrosine), d'au moins un actif biologique et d'au moins un sel minéral. Ces actifs essentiels à la vie et à la régénération des cellules épidermiques peuvent, par exemple, être choisis parmi :

- 20 acides aminés - ce sont les constituants des protéines qui jouent un rôle crucial dans la structure, le métabolisme et la physiologie des cellules : alanine, arginine, acide aspartique, acide glutamique, cystéine, glutamine, glycine, histidine, hydroxyproline, isoleucine, leucine, lysine, méthionine, phénylalanine, proline, sérine, thréonine, tryptophane, tyrosine et valine.
- 14 vitamines ou assimilé à des vitamines : acide ascorbique (C), biotine (H), pantothénate de calcium (B5), acide folique (B9), Niacine (PP ou B3), Niacinamide, pyridoxal HCl (B6), Riboflavine (B2), acétate de tocophérol (E), thiamine HCl (B1), Ethyl oléate (F Oméga 9), Ethyl linoléate (F Oméga 6), Ethyl linolénate (F Oméga 3) et inositol (B7)
- 14 actifs biologiques - ce sont des composés essentiels au fonctionnement des cellules leur servant de combustibles et permettant de les dynamiser : Adénosine, Cytosine, Guanine, thymine, adénine, adénosine phosphate (AMP), adénosine triphosphate (ATP), cholestérol, glucose, glutathion, xanthine, uracile, ADN de blé et ARN de levure.
- 8 autres actifs nécessaires à la vie cellulaire - ce sont les sels minéraux qui interviennent dans la régulation des métabolismes et des échanges cellulaires : Sulfate de Magnésium, Chlorure de Potassium, Nitrate de potassium, Sulfate de Sodium, Chlorure de Calcium, Phosphate de sodium, Chlorure de Sodium et Acétate de Sodium.

De préférence, le milieu nutricellulaire comprend les 56 actifs décrits ci-dessus.

La présente invention a donc pour objet une composition cosmétique comprenant de l'éther vanillyl-butylique et un extrait de graines de *Voandzeia subterranea*, et optionnellement un milieu nutricellulaire tel que défini ci-dessus.

Selon un mode de réalisation particulier de la composition de la présente invention, ledit milieu nutricellulaire comprend au moins une vitamine ou composé assimilé à une vitamine, au moins deux acides aminés (dont au moins la tyrosine et la phénylalanine), un actif biologique et un sel minéral. Par exemple :

- ladite au moins une vitamine ou composé assimilé à une vitamine est choisi dans le groupe constitué de : acide ascorbique (C), biotine (H), pantothénate de calcium (B5), acide folique (B9), Niacine (PP ou B3), Niacinamide, pyridoxal HCl (B6), Riboflavine (B2), acétate de tocophérol (E), thiamine HCl (B1), Ethyl oléate (F Oméga 9), Ethyl linoléate (F Oméga 6), Ethyl linolénate (F Oméga 3) et inositol (B7) ;
- lesdits au moins deux acides aminés sont choisis dans le groupe constitué de : alanine, arginine, acide aspartique, acide glutamique, cystéine, glutamine, glycine, histidine, hydroxyproline, isoleucine, leucine, lysine, méthionine, phénylalanine, proline, sérine, thréonine, tryptophane, tyrosine et valine ;
- ledit au moins un actif biologique est choisi dans le groupe constitué de : Adénosine, Cytosine, Guanine, thymine, adénine, adénosine phosphate (AMP), adénosine triphosphate (ATP), cholestérol, glucose, glutathion, xanthine, uracile, ADN de blé et ARN de levure ;
- ledit au moins un sel minéral est choisi dans le groupe constitué de : Sulfate de Magnésium, Chlorure de Potassium, Nitrate de potassium, Sulfate de Sodium, Chlorure de Calcium, Phosphate de sodium, Chlorure de Sodium et Acétate de Sodium.

Selon un mode de réalisation particulier de la composition de la présente invention, ladite composition cosmétique comprend :

- de l'éther vanillyl-butylique ;
- un extrait de graines de *Voandzeia subterranea* ; et
- un milieu nutricellulaire comprenant : acide ascorbique (C), biotine (H), pantothénate de calcium (B5), acide folique (B9), Niacine (PP ou B3), Niacinamide, pyridoxal HCl (B6), Riboflavine (B2), acétate de tocophérol (E), thiamine HCl (B1), Ethyl oléate (F Oméga 9), Ethyl linoléate (F oméga 6), Ethyl linolénate (F Oméga 3), inositol (B7), alanine, arginine, acide aspartique, acide glutamique, cystéine, glutamine, glycine, histidine, hydroxyproline, isoleucine, leucine, lysine, méthionine, phénylalanine, proline, sérine, thréonine, tryptophane, tyrosine, valine, Adénosine, Cytosine, Guanine, thymine, adénine, adénosine phosphate (AMP), adénosine triphosphate (ATP), cholestérol, glucose, glutathion, xanthine, uracile, ADN de blé, ARN de levure, Sulfate de Magnésium, Chlorure de Potassium, Nitrate de potassium, Sulfate de Sodium, Chlorure de Calcium, Phosphate de sodium, Chlorure de Sodium, et Acétate de Sodium.

Selon un mode de réalisation particulier de la composition de la présente invention, ladite composition est un sérum.

La présente invention a encore pour objet l'utilisation cosmétique non thérapeutique d'une composition cosmétique selon la présente invention, pour augmenter le flux sanguin, et régénérer les fibres de collagène et d'élastine.

La présente invention a encore pour objet l'utilisation cosmétique non thérapeutique d'une composition cosmétique selon la présente invention, pour augmenter l'épaisseur de l'épiderme et la densité du réseau de collagène du derme.

La présente invention a encore pour objet l'utilisation cosmétique non thérapeutique d'une composition cosmétique selon la présente invention, pour lutter contre le vieillissement de la peau (y compris du cuir chevelu).

La présente invention a encore pour objet l'utilisation cosmétique non thérapeutique d'une composition cosmétique selon la présente invention, pour limiter la chute des cheveux, et favoriser leur repousse.

La présente invention a encore pour objet l'utilisation cosmétique non thérapeutique d'une composition cosmétique selon la présente invention, pour retarder l'apparition de cheveux blancs.

## BREVE DESCRIPTION DES FIGURES

[Figure 1] représente l'analyse de la densité du réseau de fibres de collagène du derme papillaire dans un explant traité avec une composition selon l'invention (P) par rapport à un explant témoin non traité (T).

[Figure 2] représente l'analyse de l'épaisseur de l'épiderme dans un explant traité avec une composition selon l'invention (P) par rapport à un explant témoin non traité (T).

[Figure 3] représente l'analyse de la variation de l'épaisseur de l'épiderme dans un explant traité avec une composition selon l'invention (P) par rapport à un explant témoin non traité (T).

[Figure 4] représente l'analyse de la variation du collagène I dans le derme papillaire, à J7, dans un explant traité avec une composition selon l'invention (P) par rapport à un explant témoin non traité (T).

[Figure 5] représente l'effet d'une composition selon l'invention sur la microcirculation cutanée (peau).

[Figure 6] représente l'effet d'une composition selon l'invention sur la microcirculation cutanée (cuir chevelu).

[Figure 7] représente l'effet d'une composition selon l'invention sur la microcirculation cutanée (cuir chevelu).

[Figure 8] représente l'analyse par Visioscan de l'effet de 1 soin avec une composition selon l'invention sur la profondeur moyenne des rides.

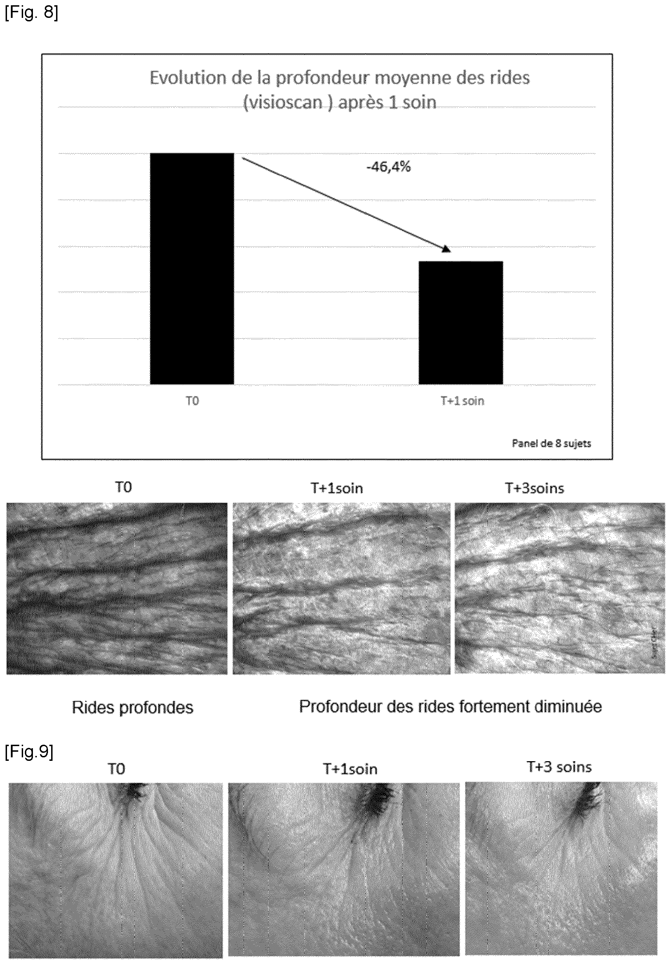

[Figure 8] représente l'analyse par appareil photo numérique de l'effet de 1 à 3 soins avec une composition selon l'invention sur la profondeur moyenne des rides.

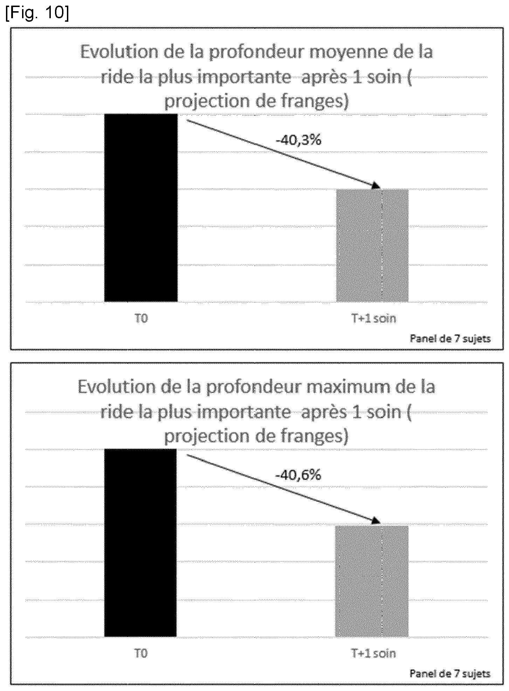

[Figure 10] représente l'analyse par l'appareil à projection de franges (PRIMOS) de la profondeur moyenne de la ride la plus importante et de la profondeur maximum de la ride la plus importante, après 1 soin avec une composition selon l'invention.

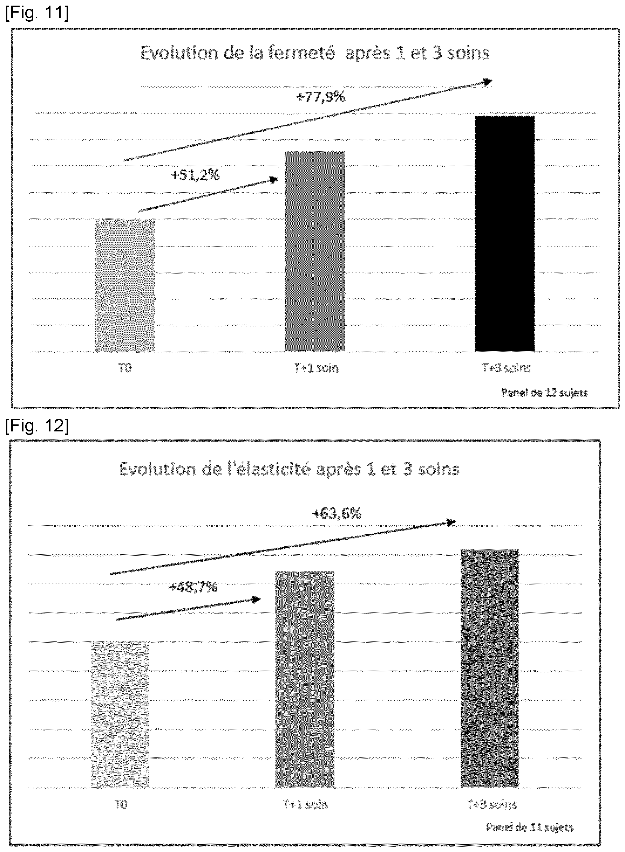

[Figure 11] représente l'analyse de la fermeté de la peau après 1 à 3 soins avec une composition selon l'invention.

[Figure 12] représente l'analyse de l'élasticité de la peau après 1 à 3 soins avec une composition selon l'invention.

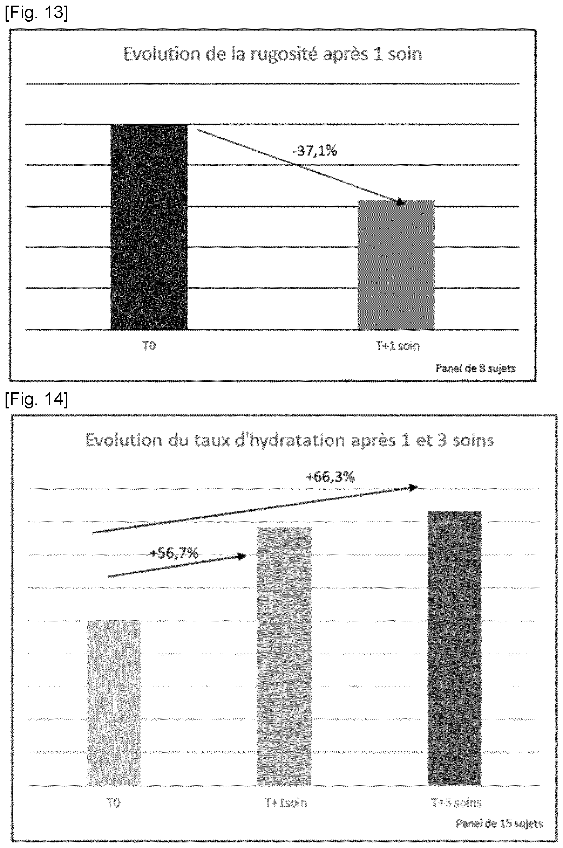

[Figure 13] représente l'analyse de la rugosité de la peau après 1 à 3 soins avec une composition selon l'invention.

[Figure 14] représente l'analyse de l'hydratation de la peau après 1 à 3 soins avec une composition selon l'invention.

## EXEMPLES

## EXEMPLE 1 : EFFET DE COMPOSITIONS COSMETIQUES SELON L'INVENTION SUR DES EXPLANTS DE PEAU HUMAINE But de l'étude

Cette étude a eu pour but d'évaluer l'activité régénérante et anti-âge après application d'une composition cosmétique selon l'invention, à partir d'explants de peau humaine ex *vivo*. La *phase ex vivo* a permis de reproduire une application de la composition sur la peau. La phase histologique a permis d'évaluer l'évolution des paramètres biologiques par coloration et immunomarquage.

L'activité a été évaluée par : des observations microscopiques après coloration au trichrome de Masson ; une mesure de l'épaisseur de l'épiderme ; un immunomarquage du collagène I.

## Matériel et Méthodes

### 1. Schéma de l'étude

| | |
|---|---|
| J0 | arrêt des explants, application composition |
| J1 | application composition |
| J2 | application composition |
| J3 | |
| J4 | arrêt des explants, application composition |
| J5 | application composition |
| J6 | application composition |
| J7 | arrêt des explants |

La composition cosmétique selon l'invention a été appliquée en topique à J0, J1, J2, J4, J5 et J6.

**2. Composition testée**

La composition de l'invention testée (concentré anti-âge Age Influx) a compris de l'éther vanillyl-butylique, un extrait de graines de *Voandzeia subterranea* et le milieu nutricellulaire à 56 actifs, telle que définie ci-dessous.

[Tableau 1]

| CONCENTRE ANTI-AGE AGE INFLUX | | |
|---|---|---|
| Nom de la matière | Nom INCI UE | % de matière |
| CETIOL V | DECYL OLEATE<br>TOCOPHEROL<br>CITRATE DE GLYCERIDES DE PALME HYDROGENES | 8 |
| CETIOL LC | COCO-CAPRYLATE/CAPRATE | 8 |
| ABIL CARE XL 80 | BIS-PEG/PPG-20/5 PEG/PPG-20/5 DIMETHICONE<br>METHOXY PEG/PPG-25/4 DIMETHICONE<br>CAPRYLIC/CAPRIC TRIGLYCERIDE<br>PENTAERYTHRITYL TETRA-DI-T-BUTYL HYDROXYHYDROCINNAMATE | 1,5 |
| DL-ALPHA TOCOPHERYL ACETATE | TOCOPHERYL ACETATE | 0,2 |
| PARFUM ZEN 14779 | PARFUM | 0,2 |
| EAU DEMINERALISEE | AQUA | 65,05 |
| SODIUM GLUCONATE | SODIUM GLUCONATE | 0,1 |
| GOMME XANTHANE FFPC | GOMME XANTHANE | 0,2 |
| (MONO)PROPYLENE GLYCOL USP/EP | PROPYLENE GLYCOL | 5 |
| SEPINOV EMT 10 | HYDROXYETHYL ACRYLATE/SODIUM ACRYLOYLDIMETHYL TAURATE COPOLYMERE<br>SORBITAN ISOSTEARATE<br>POLYSORBATE 60 | 1,5 |
| TILAMAR PDO / ZEMEA | PROPANEDIOL | 5 |
| LEXGUARD H | 1,2-HEXANEDIOL | 2 |
| EAU DEMINERALISEE | AQUA | 2 |
| MILIEU NUTRICELLULAIRE 042715 | SODIUM CHLORURE<br>GLUCOSE<br>POTASSIUM CHLORURE<br>CALCIUM CHLORURE<br>AQUA<br>ACIDE GLUTAMIQUE<br>MAGNESIUM SULFATE<br>SODIUM PHOSPHATE<br>GLUTAMINE<br>LYSINE HCL<br>ARGININE<br>LEUCINE<br>SODIUM ACETATE | 0,2 |

(suite)

| CONCENTRE ANTI-AGE AGE INFLUX | | |
| --- | --- | --- |
| Nom de la matière | Nom INCI UE | % de matière |
| | VALINE | |
| | GLYCINE | |
| | ALANINE | |
| | SODIUM SULFATE | |
| | POTASSIUM NITRATE | |
| | TYROSINE | |
| | PROLINE | |
| | ACIDE ASPARTIQUE | |
| | THREONINE | |
| | METHIONINE | |
| | ADENINE | |
| | PHENYLALANINE | |
| | SERINE | |
| | HISTIDINE | |
| | ISOLEUCINE | |
| | TRYPTOPHANE | |
| | HYDROXYPROLINE | |
| | CYSTEINE | |
| | DISODIUM ADENOSINE TRIPHOSPHATE | |
| | ARN | |
| | ADN | |
| | CHOLESTEROL | |
| | ADENOSINE PHOSPHATE | |
| | INOSITOL | |
| | ACIDE ASCORBIQUE | |
| | GLUTATHIONE | |
| | ETHYL LINOLEATE | |
| | ETHYL OLEATE | |
| | PYRIDOXINE HCL | |
| | NIACINE | |
| | NIACINAMIDE | |
| | TOCOPHEROL | |
| | THIAMINE HCL | |
| | CALCIUM PANTOTHENATE | |
| | ACIDE FOLIQUE | |
| | RIBOFLAVINE | |
| | BIOTINE | |
| | GUANINE | |
| | THYMINE | |
| | CYTOSINE | |
| | URACILE | |
| | ADENOSINE | |
| | XANTHINE | |
| EPIGENIST LS10003 | MALTODEXTRINE EXTRAIT DE GRAINES DE VOANDZEIA SUBTERRANEA | 0,5 |
| NEPHORIA BC10044 | MALTODEXTRINE | 0,1 |

(suite)

| CONCENTRE ANTI-AGE AGE INFLUX | | |
|---|---|---|
| Nom de la matière | Nom INCI UE | % de matière |
| | EXTRAIT DE BRANCHE/FRUIT/FEUILLE DE NEPHELIUM LAPPACEUM | |
| HOTFLUX | ETHER VANILLYL-BUTYLIQUE | 0,45 |

La composition a été stockée à température ambiante avant, pendant, et après la réalisation de l'étude.

## 3. Préparation des explants

L'étude a été réalisée sur du tissu cutané, obtenu à partir de résidus chirurgicaux (chirurgie esthétique) d'un donneur, dans le respect de la Déclaration d'Helsinki et de l'article L. 1243-4 du Code de la santé publique français. Cette dernière ne nécessite aucune autorisation préalable d'un comité d'éthique pour le prélèvement et l'utilisation de déchets chirurgicaux. 15 explants de $11\pm1$ mm de diamètre ont été préparés à partir d'une plastie abdominale d'une femme âgée de 46 ans de phototype II.

Les explants ont été mis en survie en milieu BEM (BIO-EC's Explants Medium) à 37°C en atmosphère humide, enrichie de 5% de $CO_2$.

## 4. Répartition des explants

Les explants ont été répartis en 3 lots comme suit :

| Lot: | Désignation/Traitement : | Nb d'explants : | Arrêt : |
|---|---|---|---|
| T0 | Témoin plastie | 3 | J0 |
| T | Témoin non traité | 6 | J4, J7 |
| P | Composition appliquée | 6 | J4, J7 |

A J0, J1, J2, J4, J5 et J6, la composition testée a été appliquée en topique, à raison de 2 $\mu$l par explant de 1 $cm^2$ (~2 $mg/cm^2$).

Les explants des lots témoin n'ont reçu aucun traitement, excepté le renouvellement du milieu. Le milieu a été renouvelé pour moitié (1 mL/puits) à J1, J4 et J6.

## 5. Prélèvements

A J0, les 3 explants du lot T0 ont été prélevés et coupés en deux. Une partie a été fixée dans une solution de formol tamponnée, et l'autre a été congelée à -80°C.

A J4 et J7, 3 explants de chaque lot ont été prélevés et traités de la même façon qu'à J0.

## 6. Traitements histologiques

Après 24 h dans le formol tamponné, les prélèvements ont été déshydratés et imprégnés en paraffine à l'aide d'un automate de déshydratation Leica PEARL. Ils ont été mis en bloc à l'aide d'une station d'enrobage Leica EG 1160.

Des coupes de 5 $\mu$m ont été réalisées à l'aide d'un microtome type Minot, Leica RM 2125 et montées sur des lames de verre histologiques Superfrost®.

Les échantillons congelés ont été découpés en sections de 7 $\mu$m d'épaisseur à l'aide d'un cryostat Leica CM 3050. Les sections ont ensuite été montées sur lames de verre Superfrost® plus.

Les observations microscopiques ont été réalisées en microscopie optique, à l'aide d'un microscope Leica type DMLB, Olympus BX43 ou Olympus BX63. Les prises de vue ont été réalisées avec une caméra Olympus DP72 ou DP74 et le logiciel CellSens.

## 6.1. Contrôle de la viabilité cellulaire (lots concernés: tous, soit 15 explants)

La viabilité cellulaire a été observée sur coupes en paraffine après coloration au trichrome de Masson variante de Goldner.

Elle a été évaluée par un examen microscopique et une mesure de l'épaisseur de l'épiderme.

Zone analysée : épiderme.

**6.2. Epaisseur de l'épiderme (lots concernés: tous, soit 15 explants)**

La mesure de l'épaisseur de l'épiderme (en $\mu$m) a été réalisée sur les images du trichrome de Masson à l'aide du logiciel CellSens.

Analyse d'images : 3 mesures par image sur 9 images, soit 27 valeurs par lot.

**6.3. Immunomarquage du collagène I (lots concernés : T0, [T, P] à J7, soit 9 explants)**

L'immunomarquage du collagène I a été réalisé à partie de coupes congelées avec un anticorps polyclonal anti-collagène I (Abcam, réf. ab138492-1001), dilué au 1:800^{ème} dans du PBS-BSA 0,3%-Tween 2 à 0,05% pendant 1 h à température ambiante et révélé en AlexaFluor AF488 (Lifetechnologies, réf A11008). Les noyaux ont été contre-colorés à l'iodure de propidium. L'immunomarquage a été évalué par un examen microscopique.

**Résultats**

**1. Résultats visuels**

Les observations ont été réalisées après coloration au trichrome de Masson variante de Goldner.

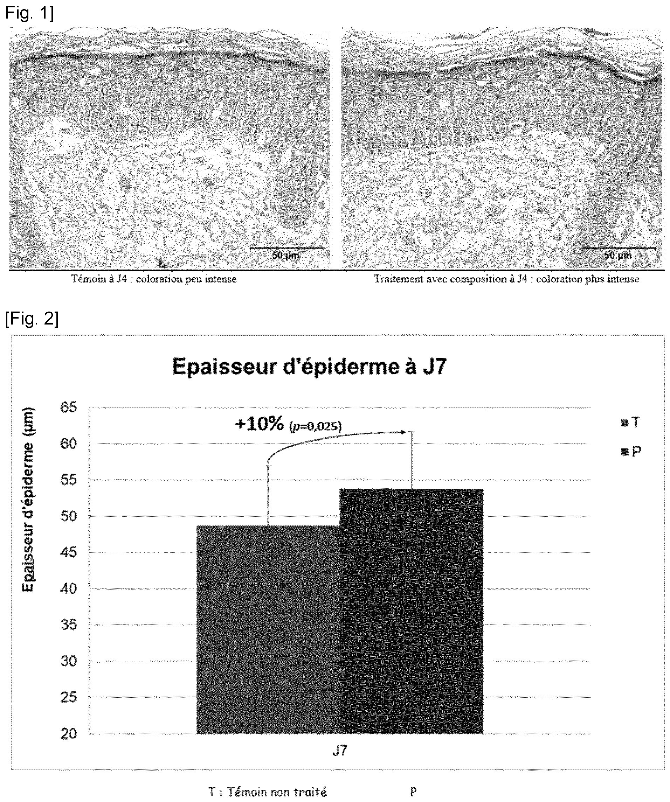

A J4, la figure 1 a montré une augmentation de la densité du réseau de fibres de collagène du derme papillaire dans l'explant traité avec la composition selon l'invention (P) par rapport à l'explant témoin non traité (T).

**2. Résultats sur l'épaisseur de l'épiderme**

La mesure de l'épaisseur de l'épiderme a été réalisée sur les images du trichrome de Masson à l'aide du logiciel CellSens.

Analyse d'image : mesure de l'épaisseur de l'épiderme, 3 mesures par image sur 9 images, soit 27 valeurs par lot.

La figure 2 a montré qu'après 6 applications, à J7, le traitement avec la composition selon l'invention a induit une augmentation significative de l'épaisseur de l'épiderme de 10% en moyenne des explants traités avec la composition selon l'invention (P) par rapport aux explants témoin non traités (T).

**3. Visualisation de l'épaisseur de l'épiderme**

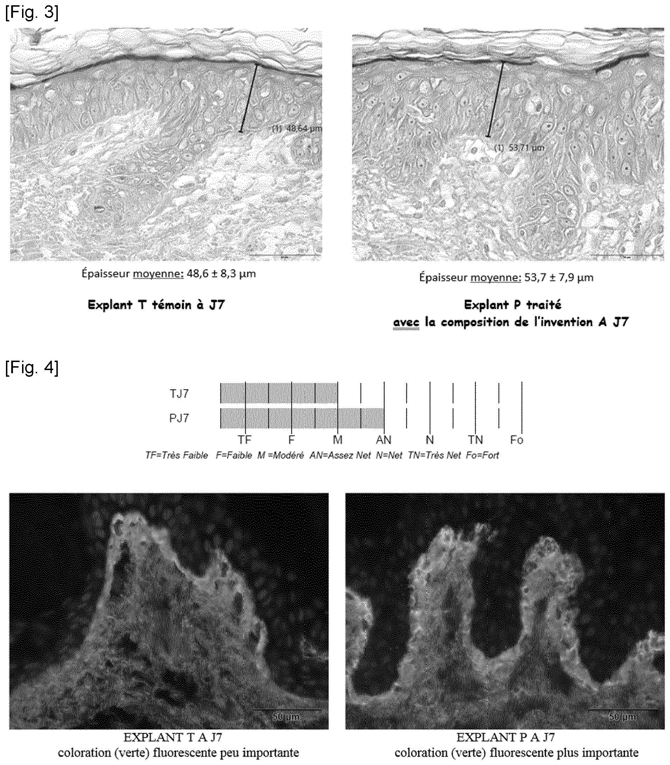

La figure 3 a montré qu'après 6 applications, à J7, le traitement avec la composition selon l'invention a induit une augmentation significative de l'épaisseur de l'épiderme de 10% dans l'explant traité avec la composition selon l'invention (P) par rapport à l'explant témoin non traité (T).

**4. Immunomarquage du collagène I**

Les observations microscopiques ont été réalisées après immunomarquage du collagène I dans le derme papillaire.

La figure 4 a montré que la composition de l'invention a induit une augmentation significative du collagène I dans le derme papillaire, à J7, dans l'explant traité avec la composition selon l'invention (P) par rapport à l'explant témoin non traité (T).

**Conclusions**

La composition selon l'invention a augmenté significativement de 10% l'épaisseur de l'épiderme à J7 (contre 112 applications de l'extrait de graines de *Voandzeia subterranea* seul), augmenté la densité du réseau de collagène à J4, et augmenté le collagène I à J7. La composition selon l'invention a donc induit une activité régénérante épidermique et dermique significative. Elle a participé ainsi à l'amélioration de la qualité de l'épiderme et du derme pour retrouver l'aspect d'une peau jeune.

Grâce à cette combinaison unique d'éther vanillyl-butylique, d'extrait de graines de *Voandzeia subterranea* et de milieu nutricellulaire, la composition de l'invention agit contre toutes les manifestations du vieillissement cutané par les mécanismes suivants :

[Tableau 2]

| Ether Vanillyl-butylique | Exrait de graines de *Voandzeia subterranea* | Milieu nutricellulaire |
|---|---|---|
| - augmente localement l'afflux sanguin (cellules mieux irriguées) donc apport en oxygène et nutriments<br><br>- amélioration du métabolisme cellulaire<br>- optimise la pénétration des autres actifs | - relance l'innervation cutanée<br><br>- métabolisme cellulaire plus performant et régénération cellulaire favorisée | - stimule la régénération cutanée (peau) |

La peau ainsi traitée par une composition de la présente invention a une apparence plus jeune, plus lisse et plus ferme avec une augmentation de l'épaisseur de l'épiderme et une augmentation de densité du réseau de collagène du derme.

**EXEMPLE 2 : EFFET DE COMPOSITIONS COSMETIQUES SELON L'INVENTION**

**SUR LA MICROCIRCULATION CUTANEE**

**But de l'étude**

Cette étude a eu pour but d'évaluer l'efficacité *in vivo* sur la microcirculation cutanée de 2 compositions cosmétiques selon l'invention.

**Matériel et Méthodes**

**1. Schéma de l'étude**

L'évaluation a été prévue comme suit :

[Tableau 3]

| | T-2jours avant application | T0 | T+30 jours après application |
|---|---|---|---|
| rasage d'une zone du cuir chevelu | ■ | | |
| laser doppler sur le cuir chevelu et 1 zone sur le front | | ■ | ■ |

L'étude a été réalisée comme une étude « ouverte » où les sujets et le technicien connaissent le type de composition testée. L'étude n'est pas comparative. Les sujets servent de référence à eux-mêmes, et les résultats obtenus à T+30 minutes ont été comparés à ceux obtenus à T0.
La sélection de la zone du front pour l'application de chaque soin du visage a été déterminée au hasard pour chaque sujet.

**2. Compositions testées**

**2.1. Présentation des compositions testées**

Deux compositions cosmétiques selon l'invention ont été testées : le Concentré anti-âge Age Influx (également dénommé ci-après Sérum âge influx) tel que décrit ci-dessus, et le Concentré NewLife ci-dessous :

[Tableau 4]

| | CONCENTRE NEWLIFE | | |
|---|---|---|---|
| | **Formule 1** | | |
| Phase | Nom de la matière | Nom INCI UE | % de matière |
| 1 | EAU DEMINERALISEE | AQUA | 79,4 |
| 1 | GLYCERINE NATURELLE CODEX | GLYCERINE | 8,0 |
| 1 | EDETA BD | DISODIUM EDTA AQUA | 0,1 |
| 2 | GLUCAM E 20 | METHYL GLUCETH-20 | 2,0 |
| 2 | GOMME XANTHANE FFPC | GOMME DE XANTHANE | 0,05 |
| 3 | LEXGUARD H | 1,2-HEXANEDIOL | 1,5 |
| 3 | SYMSAVE H | HYDROXYACETOPHENONE | 0,3 |
| 4 | HOTFLUX | ETHER VANILLYL-BUTYLIQUE | 0,45 |
| 4 | MONTANOX 20 DF | POLYSORBATE 20 | 3,0 |
| | MILIEU | SODIUM CHLORURE GLUCOSE POTASSIUM CHLORURE CALCIUM CHLORURE AQUA ACIDE GLUTAMIQUE MAGNESIUM SULFATE SODIUM PHOSPHATE GLUTAMINE LYSINE HCL ARGININE LEUCINE SODIUM ACETATE VALINE GLYCINE ALANINE SODIUM SULFATE POTASSIUM NITRATE TYROSINE PROLINE ACIDE ASPARTIQUE THREONINE METHIONINE ADENINE PHENYLALANINE SERINE HISTIDINE ISOLEUCINE TRYPTOPHANE HYDROXYPROLINE | |

(suite)

| CONCENTRE NEWLIFE | | | |
|---|---|---|---|
| **Formule 1** | | | |
| Phase | Nom de la matière | Nom INCI UE | % de matière |
| 5 | NUTRICELLULAIRE 042715 | CYSTEINE<br>DISODIUM ADENOSINE<br>TRIPHOSPHATE<br>ARN<br>ADN<br>CHOLESTEROL<br>ADENOSINE PHOSPHATE<br>INOSITOL<br>ACIDE ASCORBIQUE<br>GLUTATHIONE<br>ETHYL LINOLEATE<br>ETHYL OLEATE<br>PYRIDOXINE HCL<br>NIACINE<br>NIACINAMIDE<br>TOCOPHEROL<br>THIAMINE HCL<br>CALCIUM PANTOTHENATE<br>ACIDE FOLIQUE<br>RIBOFLAVINE<br>BIOTINE<br>GUANINE<br>THYMINE<br>CYTOSINE<br>URACILE<br>ADENOSINE<br>XANTHINE | 0,2 |
| 5 | EAU DEMINERALISEE | AQUA | 2,0 |
| 6 | EPIGENIST LS10003 | MALTODEXTRINE EXTRAIT DE GRAINES DE VOANDZEIA SUBTERRANEA | 0,5 |
| 6 | EAU DEMINERALISEE | AQUA | 2,5 |
| **Formule 2** | | | |
| | EAU DEMINERALISEE | AQUA | 77,4 |
| | GLYCERINE NATURELLE CODEX | GLYCERINE | 8,0 |
| | EDETA BD | DISODIUM EDTA AQUA | 0,1 |
| | GLUCAM E 20 | METHYL GLUCETH-20 | 2,0 |
| | GOMME XANTHANE FFPC | GOMME DE XANTHANE | 0,05 |

(suite)

| Formule 2 | | | |
|---|---|---|---|
| | LEXGUARD H | 1,2-HEXANEDIOL | 1,5 |
| | SYMSAVE H | HYDROXYACETOPHENONE | 0,3 |
| | HOTFLUX | ETHER VANILLYL-BUTYLIQUE | 0,45 |
| | MONTANOX 20 DF | POLYSORBATE 20 | 3,0 |
| | MILIEU NUTRICELLULAIRE 042715 | SODIUM CHLORURE<br>GLUCOSE<br>POTASSIUM CHLORURE<br>CALCIUM CHLORURE | 0,2 |
| | | AQUA<br>ACIDE GLUTAMIQUE<br>MAGNESIUM SULFATE<br>SODIUM PHOSPHATE<br>GLUTAMINE<br>LYSINE HCL<br>ARGININE<br>LEUCINE<br>SODIUM ACETATE<br>VALINE<br>GLYCINE<br>ALANINE<br>SODIUM SULFATE<br>POTASSIUM NITRATE<br>TYROSINE<br>PROLINE<br>ACIDE ASPARTIQUE<br>THREONINE<br>METHIONINE<br>ADENINE<br>PHENYLALANINE<br>SERINE<br>HISTIDINE<br>ISOLEUCINE<br>TRYPTOPHANE<br>HYDROXYPROLINE<br>CYSTEINE<br>DISODIUM ADENOSINE TRIPHOSPHATE<br>ARN<br>ADN<br>CHOLESTEROL<br>ADENOSINE PHOSPHATE<br>INOSITOL<br>ACIDE ASCORBIQUE<br>GLUTATHIONE<br>ETHYL LINOLEATE<br>ETHYL OLEATE<br>PYRIDOXINE HCL<br>NIACINE | |

(suite)

| Formule 2 | | | |
|---|---|---|---|
| | | NIACINAMIDE<br>TOCOPHEROL<br>THIAMINE HCL<br>CALCIUM PANTOTHENATE<br>ACIDE FOLIQUE<br>RIBOFLAVINE<br>BIOTINE<br>GUANINE<br>THYMINE<br>CYTOSINE<br>URACILE<br>ADENOSINE<br>XANTHINE | |
| | EAU DEMINERALISEE | AQUA | 2,0 |
| | EPIGENIST LS10003 | MALTODEXTRINE<br>EXTRAIT DE GRAINES DE<br>VOANDZEIA<br>SUBTERRANEA | 0,5 |
| | EAU DEMINERALISEE | AQUA | 2,5 |
| | ANAGELINE BIO | AQUA<br>PROTEINE DE LUPINE<br>HYDROLYSEE<br>SODIUM BENZOATE | 2,0 |

Les compositions à tester ont été stockées dans une pièce climatisée (avec contrôle de la température) à l'abri de la lumière.

## 2.2. Application des compositions testées

L'application des compositions à tester a été réalisée par un technicien.

[Tableau 5]

| Compositions | Localisation de l'application | Fréquence de l'application | Durée de l'étude | Stockage |
|---|---|---|---|---|
| Sérum Age Influx = Concentré anti-âge Age Influx | une zone du front choisie au hasard | Une seule application | 30 minutes après application | A température ambiante |
| Lotion (Concentré NewLife) | une zone rasée de 2,25 cm$^2$ sur le cuir chevelu | | | |

L'application du sérum été réalisée une fois, en utilisant un doigtier en latex, avec un massage circulaire, jusqu'à complète pénétration, sur une surface délimitée, de 9 cm$^2$ (3cm x 3cm) sur le front, selon une répartition aléatoire. La quantité de composition devant être appliquée sur la surface délimitée correspond à 2 µl/cm$^2$ soit 18 µl de produit.

L'application de la lotion capillaire a été réalisée une fois, en utilisant un doigtier en latex, avec un massage circulaire, jusqu'à complète pénétration, sur une surface rasée du cuir chevelu de 2,25 cm$^2$ (1,5cm x 1,5cm), selon une répartition aléatoire. La quantité de composition devant être appliquée sur la surface délimitée correspond à 10 $\mu$l de produit.

## 3. Populations

10 sujets ont été sélectionnés pour cette étude. Ces sujets ont dû répondre strictement aux critères d'inclusion / de non-inclusion définis ci-dessous.

Le panel de sujets a été composé de sujets sélectionnés sur la base d'un questionnaire électronique rempli sur un ordinateur avant le début de l'étude. Ce questionnaire a fourni des détails au sujet de l'historique médical des sujets y compris les potentielles allergies ainsi que des détails sur leur routine de soins de la peau et de maquillage. Le questionnaire a également inclus certaines informations administratives concernant les sujets. Les enregistrements ont été réalisés par un technicien qualifié. Pour faire partie du panel, les sujets ont accepté de communiquer leurs données personnelles requises pour les sélections. Ils ont été invités à consulter la politique de confidentialité associée à l'étude afin de connaitre exactement leurs droits et les conditions dans lesquelles ces données seront traitées en respectant le règlement général sur la protection des données (UE) 2016/679 (GDPR).

Pour faire partie du panel, les sujets ont confirmé qu'ils répondaient aux exigences suivantes :

- être affilié à un régime de protection sociale
- ne pas être privé de liberté par décision judiciaire ou administrative ou en vertu d'un régime de protection juridique (tutelle ou curatelle) ;
- ne pas être mineur ou ne pas être en mesure d'exprimer un consentement ;
- être en mesure de comprendre le français : sujet francophone capable de lire les documents qui lui sont présentés et d'accepter ce qui lui est expliqué ;
- être disponible pour toute la durée de l'étude ;
- être motivé pour participer librement à cette étude ;
- être prêt à suivre la procédure d'application des compositions testées dans son intégralité ;
- être capable de justifier d'une adresse permanente ;
- avoir donné leur consentement libre et éclairé par une déclaration écrite selon laquelle leurs données personnelles peuvent être traitées aux fins de l'étude.

### 3.1. Critères d'inclusion

- sujets masculins ou féminins (distribution non homogène) ;
- sujets sains (pas de pathologie non stabilisée susceptible d'interférer avec l'étude, pas de symptômes dans le cadre d'un bilan exploratoire) ;
- être âgés de 18 ans et plus ;
- avoir un phototype de I à IV ;
- avoir une peau saine sur l'unité anatomique étudiée (sans eczéma, psoriasis, plaies, cicatrice inflammatoire, dermatite séborrhéique...)

3.2. Critères de non-inclusion

3.2.1. Critères standard

- pour les sujets féminins : être ou soupçonner d'être enceinte ou vouloir être enceinte ou allaiter ;
- avoir refusé de donner leur accord en ne signant pas le formulaire de consentement éclairé ;
- participer à une autre étude susceptible d'interférer avec la présente étude ;
- avoir une allergie diagnostiquée à un ou plusieurs ingrédients de produits cosmétiques ou à des aliments ou au latex ;
- avoir appliqué un produit cosmétique (y compris du maquillage) sur les zones étudiées à T+2 jours (le visage doit être nettoyé uniquement à l'eau) ;
- avoir appliqué des produits cosmétiques ou de l'eau sur les cheveux entre le soir précédant le jour de l'étude et T+2jour (les cheveux doivent être lavés avec leur shampooing habituel le soir précédant T+2 jour et rien d'autre ne doit être appliqué par la suite sur le cuir chevelu (laques, gels coiffants, après-shampooing,...).

### 3.2.2. Critères spécifiques

- avoir une peau grasse sur le visage ;
- avoir fait une coloration, une décoloration, un éclaircissement ou une permanente chez le coiffeur ou à domicile au cours de la semaine précédente ;
- avoir refusé le rasage d'une surface d'environ 2,25 cm$^2$ sur le cuir chevelu ;
- avoir des antécédents de chirurgie du cuir chevelu (transplantation de cheveux...) ;
- avoir un cuir chevelu sensible.

### 4. Acquisition par doppler laser pour l'analyse de la vascularisation

### 4.1. Acquisition des données sources

Le système PeriCam PSI est un imageur de perfusion sanguine basé sur la technologie Laser Speckle Contrast Analysis (LASCA). LASCA fournit de nouveaux moyens non invasifs d'étudier la microcirculation d'une zone de la peau et d'évaluer le flux sanguin cutané.

Les acquisitions ont été réalisées dans une chambre noire sous température contrôlée (21±1,5°C).

Une période de 20 minutes d'acclimatation dans une pièce climatisée a été respectée pour les sujets avec les mesures (température : 21±1,5°C). Les sujets ont été allongés sur un lit, sur le ventre.

Les mesures ont été réalisées sur des surfaces rasées du cuir chevelu de 2,25 cm$^2$, 2 jours avant, et sur une surface du front.

Une mesure a été prise dans chaque surface étudiée, pour chaque sujet et chaque temps d'examen.

### 4.2. Traitement des données sources

Les mesures ont été exprimées comme la moyenne de la perfusion de chaque zone étudiée. Une augmentation de la perfusion moyenne a montré une vasodilatation ou une stimulation de la microcirculation cutanée.

### 5. Programme des examens

L'effet des compositions testées a été évalué sur une période de 30 minutes, comme suit :

A T-2 jours : signature de la feuille d'information, obtention du formulaire de consentement, vérification des critères d'inclusion/de non-inclusion, et rognage d'une zone du cuir chevelu (2,25 cm$^2$).

A T0 : acclimatation, vérification des restrictions, mesure par laser doppler sur les zones de rasage et surfaces du front ; application par un technicien des 3 compositions.

A T immédiat après application (T+30 minutes) : notification des événements indésirables après application, mesure par laser doppler sur les zones de rasage et les surfaces du front, fin de l'étude.

### 6. Analyse des données techniques

Les résultats ont compris : des valeurs brutes pour chaque sujet à chaque temps de mesure, déviations par rapport à T0 pour chaque sujet à chaque temps de mesure (T+30 minutes - T0), les moyennes, médianes, maxima, minima et écarts-types des valeurs brutes et des différences par rapport à T0 obtenues pour l'ensemble du panel, le nombre et le pourcentage de sujets présentant une amélioration.

Les logiciels utilisés pour l'analyse statistique : PAST version 1.37, SigmaStat version 3.0.1a et Microsoft Excel (utilisé uniquement pour le t-test de Student et le test de Fisher).

Tout d'abord, la normalité des distributions a été vérifiée à l'aide du test de Shapiro-Wilk avec un niveau de signification fixé à 1% pour chaque composition. Ensuite, l'analyse statistique de l'évolution du paramètre de l'étude pour chaque composition a été réalisée avec le t-test de Student (si la normalité des distributions était confirmée) ou avec le test de Wilcoxon (si la normalité était confirmée).

### Résultats

Mesure laser doppler 30 minutes après application d'une compositions cosmétique de l'invention.

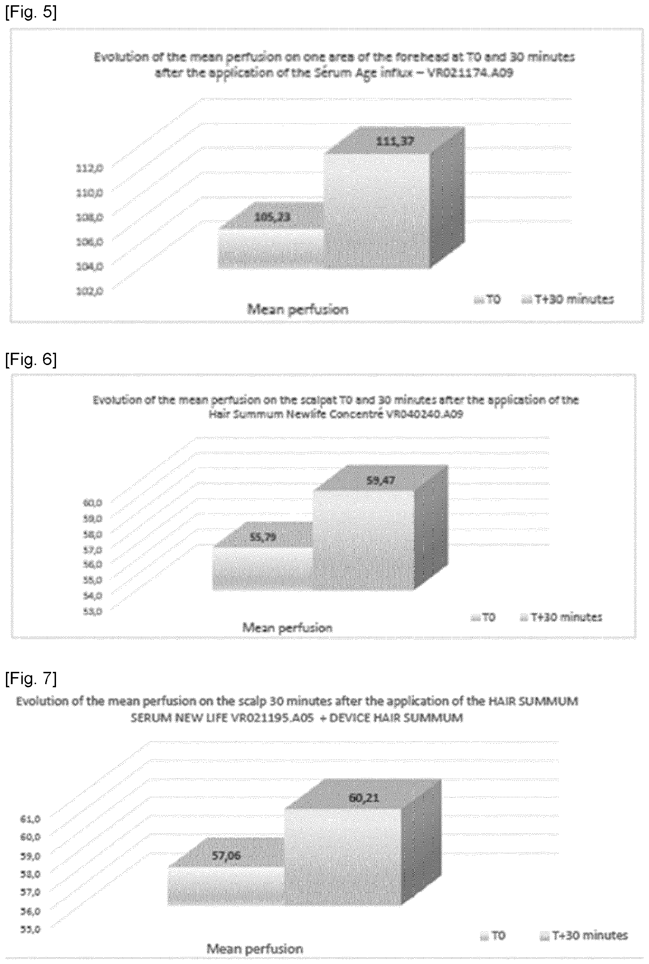

La figure 5 a montré que l'application du sérum Age Influx a augmenté la perfusion moyenne de 5,8%. L'augmentation de la perfusion moyenne traduit une vasodilatation ainsi qu'une stimulation de la microcirculation cutanée. Le Sérum Age Influx (= Concentré anti-âge Age Influx) a donc induit une augmentation significative de la microcirculation cutanée.

La figure 6 a montré que l'application du Concentré NewLife, a augmenté la perfusion moyenne de 6,6%. L'augmentation de la perfusion moyenne traduit une vasodilatation ainsi qu'une stimulation de la microcirculation cutanée. Le concentré New Life a donc induit une augmentation significative de la microcirculation du cuir chevelu.

## EXEMPLE 3 : EFFET DE COMPOSITIONS COSMETIQUES DE L'INVENTION SUR LES FOLLICULES PILEUX

### But de l'étude

Cette étude a pour but une évaluation clinique et comparaison de l'efficacité anti-chute d'une composition cosmétique selon l'invention (après-shampoing, sérum NewLife) par rapport à l'association de ladite composition et d'un appareil de soin capillaire doté d'une électrode vibrante et d'un peigne ionisant (Demande de Brevet FR 2202529) pour son application sur un panel de 40 sujets masculins et féminins après applications répétées pendant 84 jours par : trichoscan pour l'analyse du cycle de croissance des cheveux, laser doppler sur le cuir chevelu, questionnaire d'auto-évaluation et microscopie électronique à balayage.

### Matériel et Méthodes

### 1. Schéma de l'étude

L'évaluation a été prévue comme suit :

[Tableau 6]

|  | T0 | T+2 | T+30 min | T+28j | T+56j | T+84j | T+86j |
|---|---|---|---|---|---|---|---|
| Rasage (zone de 1 cm$^2$ sur le cuir chevelu) | ■ |  |  | ■ | ■ | ■ |  |
| Trichoscan pour analyse du cycle de croissance du cheveu |  | ■ |  |  |  |  | ■ |
| Laser doppler sur le cuir chevelu (10 sujets) |  | ■ | ■ |  |  |  |  |
| Microscopie à balayage électronique (3 sujets) |  | ■ |  |  |  | ■ |  |
| Questionnaire d'évaluation personnelles |  |  |  |  |  |  | ■ |
| NB : T+30 minutes après application de l'après-shampoing, correspond à 20 minutes après rinçage. | | | | | | | |

Pour le groupe testant l'après-shampoing selon l'invention et l'appareil de soin capillaire (Demande de Brevet FR 2202529), un soin par semaine est réalisé par un coiffeur. Le calendrier d'application est réalisé comme suit :

1ère application : J0
2ème application : J8
3ème application : J15
4ème application : J16
5ème application : J23
6ème application : J30
7ème application : J35
8ème application : J49
9ème application : J51
10ème application : J58
11ème application : J65
12ème application : J72

L'étude est réalisée comme une étude « ouverte » où les sujets et le technicien connaissent le type de composition testée. L'étude est une étude comparative où les résultats obtenus après application de l'après-shampoing seul sont comparés avec les résultats obtenus après l'application de l'après-shampoing avec l'appareil de soin capillaire. Les sujets ne servent pas de référence à eux-mêmes.

## 2. Composition testée

### 2.1. Présentation des compositions testées

La composition selon l'invention testée (en tant qu'après-shampoing avec rinçage) est le sérum NewLife (formule 1) tel que décrit ci-dessous, appliquée seule sur le cuir chevelu ou à l'aide de l'appareil de soin capillaire (Demande de Brevet FR 2202529).

[Tableau 7]

| SERUM NEWLIFE | | |
|---|---|---|
| **Formule 1** | | |
| Nom de la matière | Nom INCI UE | % de matière |
| SP INCROQUAT BEHENYL TMC-25 MBAL-PA-(MH) | ALCOOL CETEARYLIQUE BEHENTRIMONIUM CHLORIDE | 6 |
| DL-ALPHA TOCOPHERYL ACETATE | TOCOPHERYL ACETATE | 0,2 |
| SP CRODAMOL SS MBAL-PA-(RB) | CETYL ESTERS | 2 |
| BUTYLENE GLYCOL 1,3 | BUTYLENE GLYCOL | 10 |
| EAU DEMINERALISEE | AQUA | 66,37 |
| MERQUAT 2200 | POLYQUATERNIUM-7 | 0,3 |
| NATROSOL 250 M PHARMA | HYDROXYETHYLCELLULOSE | 0,3 |
| H.DE JOJOBA BIO | SIMMONDSIA CHINENSIS OIL | 3 |
| BELSIL ADM 6057E | AQUA AMODIMETHICONE TRIDECETH-10 CETRIMONIUM CHLORIDE | 4 |
| D PANTHENOL 50 P | PROPYLENE GLYCOL PANTHENOL | 0,5 |
| PARFUM COLORATION DIVINE MOD 1-1816280 | PARFUM HEXYL CINNAMAL LINALOOL CITRONELLOL ALPHA-ISOMETHYL IONONE | 1,5 |
| EAU DEMINERALISEE | AQUA | 1 |
| CHLORHEXIDINE DIGLUCONATE SOL. | AQUA CHLORHEXIDINE DIGLUCONATE | 0,35 |
| ACIDE LACTIQUE | ACIDE LACTIQUE AQUA | 0,04 |
| HOTFLUX | ETHER VANILLYL-BUTYLIQUE | 0,5 |
| SODIUM CARBONATE CRIST. 10H$_2$O | SODIUM CARBONATE | 0,04 |
| EAU DEMINERALISEE | AQUA | 0,2 |
| EPIGENIST LS10003 | MALTODEXTRINE EXTRAIT DE GRAINES DE VOANDZEIA SUBTERRANEA | 0,5 |
| EAU DEMINERALISEE | AQUA | 2 |
| MILIEU NUTRICELLULAIRE 042715 | SODIUM CHLORURE GLUCOSE POTASSIUM CHLORURE | 0,2 |

(suite)

| SERUM NEWLIFE | | |
|---|---|---|
| **Formule 1** | | |
| Nom de la matière | Nom INCI UE | % de matière |
| | CALCIUM CHLORURE | |
| | AQUA | |
| | ACIDE GLUTAMIQUE | |
| | MAGNESIUM SULFATE | |
| | SODIUM PHOSPHATE | |
| | GLUTAMINE | |
| | LYSINE HCL | |
| | ARGININE | |
| | LEUCINE | |
| | SODIUM ACETATE | |
| | VALINE | |
| | GLYCINE | |
| | ALANINE | |
| | SODIUM SULFATE | |
| | POTASSIUM NITRATE | |
| | TYROSINE | |
| | PROLINE | |
| | ACIDE ASPARTIQUE | |
| | THREONINE | |
| | METHIONINE | |
| | ADENINE | |
| | PHENYLALANINE | |
| | SERINE | |
| | HISTIDINE | |
| | ISOLEUCINE | |
| | TRYPTOPHANE | |
| | HYDROXYPROLINE | |
| | CYSTEINE | |
| | DISODIUM ADENOSINE TRIPHOSPHATE | |
| | ARN | |
| | ADN | |
| | CHOLESTEROL | |
| | ADENOSINE PHOSPHATE | |
| | INOSITOL | |
| | ACIDE ASCORBIQUE | |
| | GLUTATHIONE | |
| | ETHYL LINOLEATE | |
| | ETHYL OLEATE | |
| | PYRIDOXINE HCL | |
| | NIACINE | |
| | NIACINAMIDE | |
| | TOCOPHEROL | |
| | THIAMINE HCL | |
| | CALCIUM PANTOTHENATE | |
| | ACIDE FOLIQUE | |
| | RIBOFLAVINE | |
| | BIOTINE | |

(suite)

| SERUM NEWLIFE | | |
|---|---|---|
| **Formule 1** | | |
| Nom de la matière | Nom INCI UE | % de matière |
| | GUANINE<br>THYMINE<br>CYTOSINE<br>URACILE<br>ADENOSINE<br>XANTHINE | |
| EAU DEMINERALISEE | AQUA | 1 |
| **Formule 2** | | |
| Nom de la matière | Nom de la matière | Nom de la matière |
| SP INCROQUAT BEHENYL TMC-25 MBAL-PA-(MH) | ALCOOL CETEARYLIQUE<br>BEHENTRIMONIUM CHLORURE | 6 |
| DL-ALPHA TOCOPHERYL ACETATE | TOCOPHERYL ACETATE | 0,2 |
| SP CRODAMOL SS MBAL-PA-(RB) | CETYL ESTERS | 2 |
| BUTYLENE GLYCOL 1,3 | BUTYLENE GLYCOL | 10 |
| EAU DEMINERALISEE | AQUA | 64,37 |
| MERQUAT 2200 | POLYQUATERNIUM-7 | 0,3 |
| NATROSOL 250 M PHARMA | HYDROXYETHYLCELLULOSE | 0,3 |
| H.DE JOJOBA BIO | SIMMONDSIA CHINENSIS OIL | 3 |
| BELSIL ADM 6057E | AQUA<br>AMODIMETHICONE<br>TRIDECETH-10<br>CETRIMONIUM CHLORURE | 4 |
| D PANTHENOL 50 P | PROPYLENE GLYCOL<br>PANTHENOL | 0,5 |
| PARFUM COLORATION DIVINE MOD 1-1816280 | PARFUM<br>HEXYL CINNAMAL<br>LINALOOL<br>CITRONELLOL<br>ALPHA-ISOMETHYL IONONE | 1,5 |
| EAU DEMINERALISEE | AQUA | 1 |
| CHLORHEXIDINE DIGLUCONATE SOL. | AQUA<br>CHLORHEXIDINE DIGLUCONATE | 0,35 |
| ACIDE LACTIQUE | ACIDE LACTIQUE<br>AQUA | 0,04 |
| HOTFLUX | ETHER VANILLYL-BUTYLIQUE | 0,5 |
| SODIUM CARBONATE CRIST. 10H2O | SODIUM CARBONATE | 0,04 |
| EAU DEMINERALISEE | AQUA | 0,2 |
| EPIGENIST LS10003 | MALTODEXTRINE<br>EXTRAIT DE GRAINES DE VOANDZEIA SUBTERRANEA | 0,5 |

(suite)

| Formule 2 | | |
|---|---|---|
| Nom de la matière | Nom de la matière | Nom de la matière |
| EAU DEMINERALISEE | AQUA | 2 |
| MILIEU NUTRICELLULAIRE 042715 | SODIUM CHLORURE<br>GLUCOSE<br>POTASSIUM CHLORURE<br>CALCIUM CHLORURE<br>AQUA<br>ACIDE GLURAMIQUE<br>MAGNESIUM SULFATE<br>SODIUM PHOSPHATE<br>GLUTAMINE<br>LYSINE HCL<br>ARGININE<br>LEUCINE<br>SODIUM ACETATE<br>VALINE<br>GLYCINE<br>ALANINE<br>SODIUM SULFATE<br>POTASSIUM NITRATE<br>TYROSINE<br>PROLINE<br>ACIDE ASPARTIQUE<br>THREONINE<br>METHIONINE<br>ADENINE<br>PHENYLALANINE<br>SERINE<br>HISTIDINE<br>ISOLEUCINE<br>TRYPTOPHANE<br>HYDROXYPROLINE<br>CYSTEINE<br>DISODIUM ADENOSINE TRIPHOSPHATE<br>ARN<br>ADN<br>CHOLESTEROL<br>ADENOSINE PHOSPHATE<br>INOSITOL<br>ACIDE ASCORBIQUE<br>GLUTATHIONE<br>ETHYL LINOLEATE<br>ETHYL OLEATE<br>PYRIDOXINE HCL<br>NIACINE<br>NIACINAMIDE<br>TOCOPHEROL<br>THIAMINE HCL<br>CALCIUM PANTOTHENATE | 0,2 |

(suite)

| Formule 2 | | |
|---|---|---|
| Nom de la matière | Nom de la matière | Nom de la matière |
| | ACIDE FOLIQUE RIBOFLAVINE BIOTINE GUANINE THYMINE CYTOSINE URACILE ADENOSINE XANTHINE | |
| EAU DEMINERALISEE | AQUA | 1 |
| ANAGELINE BIO | AQUA PROTEIN DE LUPIN HYDROLYSEE SODIUM BENZOATE | 2 |

Les produits sont stockés dans une pièce climatisée (avec contrôle de la température) à l'abri de la lumière.

## 2.2. Application des compositions testées

L'application de la composition à tester est réalisée par un technicien.

[Tableau 8]

| Composition testée et appareil de soin capillaire | Zone de l'application | Fréquence de l'application | Durée de l'étude | stockage |
|---|---|---|---|---|
| Après-shampoing appliqué seul (sérum New Life) | Cuir chevelu et cheveux | 3 fois/semaine | 84 jours | Température ambiante |
| Après-shampoing (sérum New Life) appliqué avec l'appareil de soin capillaire | Cuir chevelu et cheveux | 2 fois/semaine pour l'après-shampoing appliqué seul et 1 fois/semaine application de l'après-shampoing à l'aide de l'appareil de soin capillaire | 84 jours | Température ambiante |

Groupe de l'après-shampoing seul :

Durant l'étude, le sujet doit utiliser un shampoing neutre à la maison, avant l'application de l'après-shampoing. L'application de l'après-shampoing est réalisée 3 fois par semaine. Après le shampoing : Appliquer la quantité nécessaire d'après-shampoing (environ 5-10 ml) sur les cheveux humides et essorés avec une serviette ; Masser légèrement le cuir chevelu pour faire pénétrer le produit et laisser agir pendant 2-3 minutes ; Rincer abondamment à l'eau chaude.

Groupe de l'après-shampoing appliqué avec l'appareil de soin capillaire :

Une application par semaine de l'après-shampoing à l'aide de l'appareil de soin capillaire est réalisée par un coiffeur. Après le shampoing (shampoing neutre usuel du sujet, 5 minutes) : Appliquer la quantité nécessaire d'après-shampoing (environ 5-10 ml) sur les cheveux humides et essorés avec une serviette, raie par raie ; Masser légèrement le cuir chevelu avec l'électrode vibrante de l'appareil de soin capillaire en réalisant des mouvements circulaires locaux depuis le front jusqu'à la nuque (2x4 minutes) puis Peigner mèche par mèche jusqu'aux pointes la chevelure avec le peigne ionisant de l'appareil de soin capillaire (2x3 minutes) ; Rincer abondamment à l'eau froide. Les deux autres applications par semaine de l'après-shampoing (après shampoing neutre usuel) sont réalisées par le sujet à domicile (cf. groupe de l'après-shampoing seul).

## 3. Population

40 sujets sont sélectionnés pour cette étude : 20 sujets sont testés avec l'après-shampoing uniquement, et 20 sujets sont testés avec l'après-shampoing selon l'invention et l'appareil de soin capillaire (Demande de Brevet FR 2202529).

Parmi les 20 sujets testant l'après-shampoing selon l'invention et l'appareil de soin capillaire, seulement 10 sujets participent aux mesures par laser doppler sur le cuir chevelu 30 minutes après la première application.

Parmi les 20 sujets de chaque groupe, un échantillon d'une mèche de cheveu est prélevé sur 3 sujets féminins aux cheveux abîmés par groupe.

Ces sujets doivent répondre strictement aux critères d'inclusion / de non-inclusion définis ci-dessous.

Le panel de sujets a été composé de sujets sélectionnés sur la base d'un questionnaire électronique rempli sur un ordinateur avant le début de l'étude. Ce questionnaire a fourni des détails au sujet de l'historique médical des sujets y compris les potentielles allergies ainsi que des détails sur leur routine de soins de la peau et de maquillage. Le questionnaire a également inclus certaines informations administratives concernant les sujets. Les enregistrements ont été réalisés par un technicien qualifié. Pour faire partie du panel, les sujets ont accepté de communiquer leurs données personnelles requises pour les sélections. Ils ont été invités à consulter la politique de confidentialité associée à l'étude afin de connaitre exactement leurs droits et les conditions dans lesquelles ces données seront traitées en respectant le règlement général sur la protection des données (UE) 2016/679 (GDPR).

Pour faire partie du panel, les sujets ont confirmé qu'ils répondaient aux exigences suivantes :

- être affilié à un régime de protection sociale
- ne pas être privé de liberté par décision judiciaire ou administrative ou en vertu d'un régime de protection juridique (tutelle ou curatelle) ;
- ne pas être mineur ou ne pas être en mesure d'exprimer un consentement ;
- être en mesure de comprendre le français : sujet francophone capable de lire les documents qui lui sont présentés et d'accepter ce qui lui est expliqué ;
- être disponible pour toute la durée de l'étude ;
- être motivé pour participer librement à cette étude ;
- être prêt à suivre la procédure d'application des compositions testées dans son intégralité ;
- être capable de justifier d'une adresse permanente ;
- avoir donné leur consentement libre et éclairé par une déclaration écrite selon laquelle leurs données personnelles peuvent être traitées aux fins de l'étude.

### 3.1. Critères d'inclusion

### 3.1.1. Critères standard

- sujets masculins ou féminins (distribution non homogène) ;
- sujets sains (pas de pathologie non stabilisée susceptible d'interférer avec l'étude, pas de symptômes dans le cadre d'un bilan exploratoire) ;
- être âgés de 20 à 60 ans ;
- avoir un phototype de I à IV ;
- avoir une peau saine sur l'unité anatomique étudiée (sans eczéma, psoriasis, plaies, cicatrice inflammatoire, dermatite séborrhéique...)

### 3.1.2. Critères spécifiques

- avoir une longueur minimum de cheveu de 3-4 cm ;
- avoir fait un shampoing et n'avoir rien appliqué d'autre par la suite sur le cuir chevelu (laques, gels coiffants,...) le matin du premier jour de l'étude ;
- pour les sujets masculins : présenter une perte de cheveu chronique / alopécie d'origine androgénétique correspondant à la classe III, IIIvertex, IV, V ou VI selon l'échelle de Norwood Hamilton ;
- pour les sujets féminins : présenter une perte de densité capillaire.

### 3.2. Critères de non-inclusion

### 3.2.1. Critères standard

- pour les sujets féminins : être ou soupçonner d'être enceinte ou vouloir être enceinte ou allaiter ;
- avoir refusé de donner leur accord en ne signant pas le formulaire de consentement éclairé ;
- participer à une autre étude susceptible d'interférer avec la présente étude ;
- avoir débuter, changer ou stopper un traitement pour le diabète ou pour des problèmes de thyroïde dans les 6 mois précédent l'étude ;
- avoir une allergie diagnostiquée à un ou plusieurs ingrédients de produits cosmétiques ou à des aliments ;
- refuser de suivre les critères d'inclusion pendant l'étude ;
- ne pas utiliser d'autres produits cosmétiques à visée anti-chute que l'après-shampoing test sur le cuir chevelu (les produits de coiffure, les shampooings neutres habituels, les masques... sont autorisés).

### 3.2.2. Critères spécifiques

- avoir un cuir chevelu sensible ou des pellicules ou une peau atopique ;
- avoir présenté un facteur d'effluvium télogène dans les trois mois précédant le début de l'étude (stress sévère, traumatisme psychologique, fièvre élevée prolongée pendant quelques jours, régime hypocalorique, chirurgie, hémorragie,...) ;
- avoir refusé le rasage d'une surface d'environ 2,25 cm$^2$ sur le cuir chevelu ;
- avoir des antécédents de chirurgie du cuir chevelu (transplantation de cheveux...) ;

**Dans les 6 mois :**

- avoir suivi l'un des traitements spécifiques suivants : Minoxidil (local) ou Finastéride (Propecia®), Dutastéride (systémique), médicament anti-androgène ;
- avoir suivi l'un des traitements suivants connus pour provoquer une perte de cheveux : rétinoïdes, anti-coagulants, anti-convulsivants, chimiothérapie, bêtabloquants, anti-inflammatoires non-stéroïdiens de manière prolongée, stéroïdes oraux ou injectables... ;

**Dans les 3 mois :**

- avoir suivi un traitement local ou systémique contre la perte de cheveux (Biotine, Panthénol, acides aminés soufrés, vitamine B6, zinc, Anastim®,...) ;
- avoir utilisé un complément alimentaire pour la perte de cheveux ;
- avoir suivi un traitement avec du fer ;
- pour les sujets féminins : avoir modifié sa contraception hormonale.

**Dans les 15 jours :**

- avoir appliqué un produit cosmétique avec une revendication anti-chute de cheveux
- shampoing...) ;
- avoir réalisé des coiffures traumatisantes susceptibles d'endommager les cheveux ;
- avoir brossé les cheveux trop à chaud ou trop fréquemment ;
- avoir utilisé de nouveaux traitements locaux ou des produits cosmétiques sur le cuir chevelu ;
- respect de l'obligation suivante pendant l'étude : faire obligatoirement un shampoing le matin du jour des mesures et ne rien appliquer d'autre par la suite sur le cuir chevelu (laques, gels coiffants,...) ;

- non-respect des restrictions suivantes durant l'étude : ne pas commencer un nouveau médicament, ne pas utiliser un nouveau traitement local ou un produit cosmétique sur le cuir chevelu, ne pas utiliser un shampoing autre qu'un shampoing neutre durant l'étude avec une fréquence habituelle, ne pas effectuer de teinture dans la semaine précédant les mesures, ne pas effectuer de décoloration ou de permanente, ne pas effectuer de coiffure traumatique qui pourrait endommager les cheveux, ne pas réaliser de brossage trop à chaud ou avec trop de fréquence, ne pas couper les cheveux sans conserver une longueur minimum de 3-4 cm, ne pas changer de coiffure tout au long de l'étude, pour les sujets féminins : ne pas débuter, changer ou arrêter un traitement hormonal.

Toutes les restrictions présentées ci-dessus doivent être suivies durant l'étude. Ces restrictions sont également présentées dans le formulaire d'information donné au sujet avant le début de l'étude.

## 4. Procédure de l'étude

### 4.1. Trichoscan pour l'analyse du cycle de croissance du cheveu

Le trichoscan est une technique non invasive qui permet l'étude *in vivo* du cycle de croissance du cheveu composé de 3 cycles successifs :

Phase anagène (croissance) : C'est le nom de la période de croissance d'un follicule pileux. La phase anagène des follicules pileux du cuir chevelu dure généralement de 3 à 5 ans.

Phase catagène (intermédiaire) : A la fin de la période de croissance, les follicules pileux se préparent pour la phase de repos. Cette période transitoire du follicule pileux de la croissance au repos est appelée la phase catagène. Cette phase du cycle de croissance du cheveu dure environ 1 à 2 semaines. Durant la phase catagène, les parties profondes du follicule pileux commencent à se dégrader. Phase télogène (repos ou perte) : C'est la période de repos du follicule pileux. Elle dure généralement de 3 à 4 mois et à la fin de cette période les cheveux les plus anciens qui ont finis leur vie tombent et les cheveux les plus récents commencent leur croissance.

Cette technique consiste à obtenir des photographies de résolution élevée de petites surfaces rasée du cuir chevelu (environ 1 cm$^2$), dans des conditions d'éclairage tout à fait reproductibles. Pour chaque point cinétique de l'étude, les photographies sont réalisées avec un décalage de 2 jours pour permettre la croissance des cheveux, c'est-à-dire après la période pendant laquelle les cheveux en phase anagène produisent un segment de cheveux et poussent d'environ 0,7 mm.

L'évaluation est réalisée sous une température contrôlée (température : 21±1,5°C). Les sujets doivent avoir lavé, brossé et peigné leurs cheveux le matin du jour des acquisitions, selon leurs habitudes.

Pour la localisation de la zone à étudier, le sujet est assis, le dos face au technicien. Pour l'acquisition de la photographie, le sujet est allongé sur le dos.

Une zone du cuir chevelu est choisie sur le vertex. Le choix de la zone est variable entre les sujets. Sur la zone étudiée, les cheveux sont coupés et taillés de près, sur une surface d'environ 1 cm$^2$, en utilisant un gabarit. De plus, en cas de cheveux blancs ou naturellement blonds, une coloration noire des cheveux est réalisée sur la zone étudiée à T+2 jours et T+86 jours. Un rasage peut être effectué à un ou plusieurs moments intermédiaires, en fonction de la durée de l'étude, afin de garder visibles les zones étudiées.

Afin d'assurer une bonne reproductibilité des conditions d'acquisition, les photographies du cuir chevelu sont réalisées en respectant une distance, un éclairage et agrandissement standardisés. En outre, les photographies prises à chaque moment de la cinétique sont comparées à la photographie prise au départ (T0). Les acquisitions du trichoscan sont réalisées à T+2 jours (pour la mesure de référence) et à T+86 jours.

Le pourcentage de cheveux est compté dans la phase anagène et dans la phase télogène. A partir de ce compte, les calculs suivants sont réalisés : ratio entre le % de cheveux en phase anagène et le % de cheveux en phase télogène : AIT. Seul le paramètre le plus pertinent est soumis à une analyse statistique. Afin de montrer un effet du produit testé sur le cycle des cheveux, une augmentation du ratio A/T est attendue.

### 4.2. Acquisition par laser doppler

Le système PeriCam PSI est un imageur de perfusion sanguine basé sur la technologie Laser Speckle Contrast Analysis (LASCA). LASCA fournit de nouveaux moyens non invasifs d'étudier la microcirculation d'une zone de la peau et d'évaluer le flux sanguin cutané.

Les acquisitions sont réalisées dans une chambre noire sous température contrôlée (21±1,5°C).

Une période de 20 minutes d'acclimatation dans une pièce climatisée a été respectée pour les sujets avec les mesures (température : 21±1,5°C). Les sujets ont été allongé sur un lit, sur le ventre.

Les mesures sont réalisées sur des surfaces rasées de 1,55 cm$^2$, 2 jours avant. Une mesure est prise dans chaque surface étudiée, pour chaque sujet et chaque temps d'examen. Les mesures sont exprimées comme la moyenne de la perfusion de la zone étudiée. Une augmentation de 5.5% (Fig. 7) de la perfusion moyenne montre une vasodilatation ou une stimulation de la microcirculation cutanée.

### 4.3. Microscopie électronique à balayage (SEM)

L'étude du vieillissement des cheveux peut être réalisée par SEM. Cette méthode (qui peut être utilisée *in vitro* ou *in vivo*) permet d'obtenir des images à haute résolution de la topographie de la surface du cheveu. Ces mesures de la topographie de la surface du cheveu basées sur les interactions entre les électrons et la matière sont réalisées en utilisant un microscope électronique à balayage avec une émission de champ (ZEISS DSM 982 - Sonde EDS NORAN - Université médicales de Tours). Un total de 12 mèches de cheveux sont collectés pour analyse par SEM : A T0 et T+84 jours, une mèche de cheveux est prélevée pour 3 sujets féminins aux cheveux abimés dans 2 groupes. L'analyse est réalisée sur 20 cheveux par mèche de cheveux.

Les images obtenues n'ont qu'un but illustratif de visualisation de la topographie de la surface du cheveu. Aucun traitement des images n'est effectué.

### 4.4. Questionnaire d'auto-évaluation

Les sujets doivent remplir un questionnaire afin d'évaluer leur opinion globale et leur attitude à l'égard de l'efficacité de l'après-shampoing et de l'appareil de soin capillaire testés.

### 5. Calendrier des examens

Les effets de l'après-shampoing ou de l'après-shampoing et de l'appareil de soin capillaire (produit(s)) sont évalués sur une période de 84 jours, comme suit : **Pré-inclusion :** vérification des critères d'inclusion et de non-inclusion.

**A T0 avant l'application des produits :** signature de la feuille d'information, obtention du formulaire de consentement, vérification des critères d'inclusion et de non-inclusion par un technicien, rasage dune zone du cuir chevelu (1 cm2).

**A T+2 jours :** acclimatation, vérification des obligations/restrictions par un technicien, prélèvement des mèches de cheveux (3 sujets féminins par groupe), mesure par laser doppler (10 sujets du groupe après-shampoing appliqué avec l'appareil de soin capillaire), teinture de la zone rasée (si nécessaire), photographies digitales par trichoscan, application de l'après-shampoing et de l'appareil de soin capillaire (20 sujets), distribution de l'après-shampoing et du formulaire de demande quotidienne.

**A T+30 minutes après l'application de l'après-shampoing et de l'appareil de soin capillaire (20 minutes après rinçage de l'après-shampoing)** : mesure par laser doppler (10 sujets du groupe après-shampoing + appareil de soin capillaire), rapport des événements indésirables suite à l'application.

***Entre T+2 jours et T+28 jours, applications n°2, 3 et 4 de l'après-shampoing et de l'appareil de soin capillaire par un coiffeur et rapport des événements indésirables suite aux applications***

**A T+28 jours :** vérification des proscriptions et restrictions par le technicien, rapport des événements indésirables apparus durant l'étude, rasage de la zone du cuir chevelu (1 cm$^2$), application de l'après-shampoing et de l'appareil de soin capillaire (20 sujets), rapport des événements indésirables suite à l'application.

***Entre T+28 jours et T56 jours, applications n°6, 7 et 8 de l'après-shampoing et de l'appareil de soin capillaire par un coiffeur et rapport des événements indésirables suite aux applications***

**A T+56 jours :** vérification des proscriptions et restrictions par le technicien, rapport des événements indésirables apparus durant l'étude, rasage de la zone du cuir chevelu (1 cm$^2$), application de l'après-shampoing et de l'appareil de soin capillaire (20 sujets), rapport des événements indésirables suite à l'application.

***Entre T+56 jours et T84 jours, applications n°10, 11 et 12 de l'après-shampoing et de l'appareil de soin capillaire par un coiffeur et rapport des événements indésirables suite aux applications***

**A T+84 jours :** vérification des proscriptions et restrictions par le technicien, rapport des événements indésirables apparus durant l'étude, rasage de la zone du cuir chevelu (1 cm$^2$), application de l'après-shampoing et de l'appareil de soin capillaire (20 sujets), rapport des événements indésirables suite à l'application.

**A T+86 jours (en option) :** vérification des proscriptions et restrictions par le technicien, rapport des événements indésirables apparus durant l'étude, questionnaire d'auto-évaluation, teinture de la zone rasée (si nécessaire), photographies digitales par trichoscan, fin de l'étude.

## 6. Analyse des données et statistiques

### 6.1. Analyse des données techniques

Les résultats comprennent :

- valeurs brutes pour chaque sujet à chaque point de mesure,
- déviations par rapport à T0 pour chaque sujet à chaque temps de mesure (Tn-TO),
- moyennes, médianes, maximales, minimales et écart-types des valeurs brutes et des différences par rapport à T0 obtenues pour l'ensemble du panel,
- variations, par rapport à T0, exprimées comme un pourcentage calculé à partir des valeurs moyennes,
- nombre et pourcentage des sujets présentant une amélioration.

Les logiciels utilisés pour l'analyse statistique : PAST version 1.37, SigmaStat version 3.0.1a et Microsoft Excel (utilisé uniquement pour le t-test de Student et le test de Fisher).

Analyse statistique de l'évolution au cours du temps (Tn-TO), pour chaque groupe

Tout d'abord, la normalité des distributions est vérifiée à l'aide du test de Shapiro-Wilk avec un niveau de signification fixé à 1% pour chaque groupe. Ensuite, l'analyse statistique de l'évolution des paramètres de l'étude pour chaque composition est réalisée avec le t-test de Student (si la normalité des distributions est confirmée) ou avec le test de Wilcoxon (si la normalité est confirmée). Le niveau de signification est fixé à 5%.

Analyse statistique comparant 2 groupes à T0 et leur évolution au cours du temps à (Tn-T0)

Tout d'abord, la normalité des distributions de chaque échantillon et l'homogénéité des variances sont vérifiées à T0 et à (Tn-TO), à l'aide du test de Shapiro-Wilk avec un niveau de signification fixé à 1% et le test de Fisher avec un niveau de signification fixé à 5%, respectivement.

Si la normalité des distributions de chaque échantillon de même que l'homogénéité des variances sont confirmées, l'analyse statistique comparant les deux groupes, à T0 et à (Tn-TO), est réalisée sur chaque paramètre de l'étude en utilisant le t-test de Student pour les échantillons indépendants. Le niveau de signification est fixé à 5%. Si la normalité des distributions pour au moins un des deux échantillons ou l'homogénéité des variances est rejetée, l'analyse statistique comparant les deux groupes, à T0 et (Tn-TO), est réalisée sur chaque paramètre de l'étude en utilisant le test de Mann-Whitney. Le niveau de signification est fixé à 5%.

### 6.2. Analyse des données obtenues avec le questionnaire d'auto-évaluation

L'analyse des questions qualitatives consiste à compter le nombre de réponses et puis au calcul des proportions et pourcentages des différentes réponses possibles. Pour évaluer l'efficacité et l'appréciation des produits testés, pour chaque item, deux pourcentages Z1 et Z2 sont calculés comme suit :

Z1 = opinion favorable (i.e. « complètement d'accord » + « plutôt d'accord »)

Z2 = opinion défavorable (i.e. « complètement en désaccord » + « plutôt en désaccord »)

La différence statistique des fréquences (%) entre les opinions favorables et défavorables est évaluée au moyen du test de Chi-2 avec un niveau de signification fixé à 5%.

Pour la question concernant la note attribuée, la moyenne ainsi que l'écart-type, le minimum et le maximum sont calculés.

### Conclusions

Les tests *in vivo* sur les cheveux montrant une augmentation des cheveux en phase anagène et une diminution des phases télogène, ainsi qu'une amélioration de la qualité de la fibre capillaire et une diminution de l'apparition des cheveux blancs. Grâce à cette combinaison unique d'éther vanillyl-butylique, d'extrait de graines de *Voandzeia subterranea* et de milieu nutricellulaire, la composition de l'invention agit contre toutes les manifestations du vieillissement du cuir chevelu/follicules pileux par les mécanismes suivants :

[Tableau 9]

| Ether Vanillyl-butylique | Exrait de graines de *Voandzeia subterranea* | Milieu nutricellulaire |
|---|---|---|
| - augmente localement l'afflux sanguin (cellules mieux irriguées) donc apport en oxygène, nutriments, facteurs de croissance<br><br>- optimise la pénétration des autres actifs<br>- stimule la différenciation des cellules du bulbe pileux<br>- limite la chute de cheveux, favorise leur repousse<br>- retarde l'apparition des cheveux blancs | - relance l'innervation cutanée<br>- métabolisme cellulaire plus performant et régénération cellulaire favorisée | - stimule la régénération cutanée (cuir chevelu)<br>- apport de tyrosine et phénylalanine |

Le cuir chevelu ainsi traité par une composition de la présente invention présente une densité de follicules pileux plus importante qu'un cuir chevelu non traité (permet de limiter la chute de cheveux et favoriser leur repousse), et un retard de l'apparition des cheveux blancs et des cheveux fortifiés

**EXEMPLE 4 : EFFET DE COMPOSITIONS COSMETIQUES SELON L'INVENTION SUR LES RIDES**

**But de l'étude**

Cette étude a eu pour but d'évaluer l'efficacité d'une composition selon l'invention telle que définie ci-dessus (Concentré anti-âge Age Influx) en tant qu'anti-rides, mais également vis-à-vis de la fermeté, l'élasticité, la rugosité, l'hydratation de la peau.

**Matériel et Méthodes**

L'étude a été réalisée pendant 3 semaines à raison d'un soin par semaine sur un panel de plusieurs sujets.

L'application de chaque soin a été réalisée en utilisant l'appareil de soin capillaire (Demande de Brevet FR 2202529) doté d'une électrode vibrolissante et d'une électrode vibrodermie, sur une surface délimitée de la peau à traiter (e.g. rides des pattes d'oie, rides du front et ride lion, sillons nasogéniens et rides d'amertume, rides du cou et de l'ovale du visage). Après démaquillage (3 minutes) et gommage léger (3 minutes), une quantité d'environ 5 ml de concentré a été appliquée sur la peau, puis l'électrode vibrolissante a été appliquée perpendiculairement à l'implantation des rides puis en mouvements circulaires en commençant par les rides du front et la ride du lion (2 minutes), les rides des pattes d'oie (2 minutes), les sillons nasogéniens et les rides d'amertume (3 minutes) pour finir par les rides du cou et l'ovale du visage (2 minutes). L'électrode vibrodermie a ensuite été appliquée suivant le même schéma que l'électrode vibrolissante dans le but d'égaliser le derme sur la peau. Les surfaces traitées ont ensuite été démaquillées pour apaiser la peau.

**1. Mesure de la profondeur des rides**

Les mesures de la profondeur moyenne des rides ont été effectuées avant (T0), après 1 soin (T+1 soin) et après 3 soins (T+3 soins), par prise de photographies obtenues au moyen (i) d'un appareil photo numérique, (ii) du système VISIOSCAN VC 98 constitué d'une caméra et d'un éclairage UVA, et d'un appareil à projection de franges (PRIMOS).

La caméra a été appliquée sur la peau et a illuminé celle-ci avec une lumière spéciale de type UVA. La caméra a photographié la zone à tester. Les images du relief cutané ont été acquises et numérisées afin d'évaluer la surface de la peau grâce au logiciel utilisé. Les images ont pu être travaillées en 3D. La caméra a ainsi pu évaluer le relief cutané et donc de calculer la profondeur des rides.

Des calculs sur la profondeur des rides ont permis d'établir l'échelle d'évaluation suivante selon l'efficacité d'un produit appliqué sur la peau.

[Tableau 10]

| Profondeur rides : | Efficacité du produit testé : |
|---|---|
| 0 < variation < 4 | Nulle |
| 4 < variation < 10 | Moyenne |
| 10 < variation < 19 | Bonne |
| variation ≥ 19 | Excellente |

## 2. Mesure de la fermeté

Les mesures de la fermeté ont été réalisées à l'aide d'un cutomètre sur l'ovale du visage de 12 sujets. Une sonde a été appliquée sur la peau sur une zone déterminée.

Le cutomètre a permis de calculer la fermeté de la peau. La peau a été aspirée de façon répétée par la sonde avec une pression d'aspiration constante, pendant un temps donné. La pression a été mesurée en Mbar / seconde. Les mesures ont été répétées 3 fois sur chaque zone afin d'obtenir une moyenne.

Des études sur la fermeté de la peau ont permis d'établir l'échelle d'évaluation suivante sur l'efficacité d'un produit :

[Tableau 11]

| Fermeté : | Efficacité du produit testé : |
|---|---|
| 0 < variation < 10 | Nulle |
| 4 < variation < 20 | Moyenne |
| 20 < variation < 30 | Bonne |
| variation ≥ 30 | Excellente |

## 3. Mesure de l'élasticité

Les mesures de l'élasticité ont été réalisées à l'aide d'un cutomètre sur l'ovale du visage de 11 sujets. Une sonde a été appliquée sur la peau sur une zone déterminée.

Le cutomètre a permis de calculer l'élasticité de la peau. La peau a été aspirée de façon répétée par la sonde avec une pression d'aspiration constante, pendant un temps donné. La pression a été mesurée en Mbar / seconde. Les mesures ont été répétées 3 fois sur chaque zone afin d'obtenir une moyenne.

Des études sur l'élasticité de la peau ont permis d'établir l'échelle d'évaluation suivante sur l'efficacité d'un produit :

[Tableau 12]

| Elasticité : | Efficacité du produit testé : |
|---|---|
| 0% < variation < 10% | Nulle |
| 10% < variation < 20% | Moyenne |
| 20% < variation < 30% | Bonne |
| variation ≥ 30% | Excellente |

## 4. Mesure de la rugosité

Les mesures de la rugosité ont été réalisée sur un panel de 8 sujets à l'aide du VISIOSCAN VC 98 d'une caméra et d'un logiciel spécifique. Une caméra a été appliquée sur la peau et a illuminé celle-ci avec une lumière spéciale de type UVA. La numérisation des images a ainsi permis d'évaluer la surface de la peau grâce au logiciel en termes de rugosité. La caméra a permis d'évaluer le relief cutané.

Des études sur la diminution de la rugosité ont permis d'établir une échelle d'évaluation suivante sur l'efficacité d'un produit :

[Tableau 13]

| Rugosité : | Efficacité du produit testé : |
|---|---|
| 0% < variation < 4% | Nulle |
| 4% < variation < 10% | Moyenne |
| 10% < variation < 19% | Bonne |
| variation ≥ 19% | Excellente |

**5. Mesure de l'hydratation**

Les mesures d'hydratation ont été réalisées à l'aide d'un *cornéomètre* sur *3 zones* du visage de 15 sujets. Le principe a été basé sur la transmission du courant proportionnellement à la quantité d'eau présente dans la peau (capacitance).

Des études sur l'hydratation ont permis d'établir une échelle d'évaluation suivante sur l'efficacité d'un produit :

[Tableau 14]

| Hydratation : | Efficacité du produit testé : |
|---|---|
| 0% < variation < 4% | Nulle |
| 4% < variation < 15% | Moyenne |
| 15% < variation < 25% | Bonne |
| variation ≥ 25% | Excellente |

**Résultats**

**1. Résultats sur la profondeur des rides**

**1.1. Données d'analyse technique par Visioscan et Primos et appareil numérique**

L'analyse par Visioscan a montré que dès le 1er soin, la profondeur moyenne des rides a significativement diminué en moyenne de 46,4% (p = 0,0001, test significatif si p<0,05 selon t-test de Student), sur un panel de 8 sujets. La profondeur des rides a été visiblement diminuée dès le 1er soin, et diminution qui s'accentue encore après 3 soins (figure 8). Cette diminution de la profondeur des rides à partir du 1er soin qui s'accentue encore après 3 soins, a été également vérifiée au moyen d'un appareil photo numérique (figure 9).

Les mesures de la profondeur moyenne de la ride la plus importante et de la profondeur maximum de la ride la plus importante ont été réalisées à l'aide de l'appareil à projection de franges (PRIMOS) sur une zone bien déterminée de la peau à tester (patte d'oie, ride du lion). L'appareil a été placé sur la zone à tester. Le principe a reposé sur la mesure du relief de la peau par déformation et déphasage des franges projetées. Dès le 1er soin, la profondeur moyenne de la ride la plus importante a diminué de 40,3% en moyenne (p=2,45E-06, test significatif si p<0,05), et la profondeur maximum de la ride la plus importante a diminué de 40,6% en moyenne (p=0,004, test significatif si p<0,05), sur un panel de 7 sujets (figure 10).

**1.2. Données d'analyse par auto-évaluation**

[Tableau 15]

| Après 1 soin: | Après 3 soins: |
|---|---|
| > 100% des sujets ont trouvé leur peau lissée. | > 100% des sujets ont trouvé leur peau lissée. |
| > 90,9 % des sujets ont trouvé leurs rides et ridules comblées. | > 85 % des sujets ont trouvé leurs rides et ridules comblées. |

(suite)

| > 86,4 % des sujets ont trouvé leurs rides et ridules atténuées. | > 80 % des sujets ont trouvé leurs rides et ridules atténuées. |
|---|---|
| > 90,9 % des sujets ont trouvé que leur peau parait visiblement plus jeune. | 90 % des sujets ont trouvé que leur peau parait visiblement plus jeune. |
| > 95,5 % des sujets ont trouvé que leur peau a retrouvé l'éclat de la peau plus jeune. | > 90% des sujets ont trouvé que leur peau a retrouvé l'éclat de la peau plus jeune. |
| > 90,9% des sujets ont trouvé leur peau raffermie. | > 90% des sujets ont trouvé leur peau raffermie. |
| > 95,5% des sujets ont trouvé leur peau hydratée. | > 100% des sujets ont trouvé leur peau hydratée. |
| > 86,4% des sujets ont trouvé leur peau régénérée. | > 85 % des sujets ont trouvé leur peau repulpée. |
| > 90,9 % des sujets ont trouvé leur peau repulpée. | > 85 % des sujets ont trouvé leur peau redensifiée (retrouve son volume). |
| > 86,4 % des sujets ont trouvé leur peau redensifiée (retrouve son volume). | > 90% des sujets ont trouvé leur peau éclatante. |
| > 86,4% des sujets ont trouvé leur peau éclatante. | |

**2. Résultats sur la fermeté**

Dès le 1er soin, une augmentation significative de la fermeté de 51,2% en moyenne (p=8,1942E-06, test significatif si p<0,05) a été obtenue, et dès 3 soins, une augmentation significative de la fermeté de 77,9% en moyenne (p=4,1214E-05, test significatif si p<0,05) a été obtenue (figure 11), sur un panel de 12 sujets.

**3. Résultats sur l'élasticité**

Dès le 1er soin, une augmentation significative de l'élasticité de 48,7% en moyenne (p=5,0873E-06, test significatif si p<0,05) a été obtenue, et dès 3 soins, une augmentation significative de l'élasticité de 63,6% en moyenne (p=2,3466E-06, test significatif si p<0,05) a été obtenue (figure 12), sur un panel de 11 sujets.

**4. Résultats sur la rugosité**

Dès le 1er soin, une diminution significative de la rugosité de 37,1% en moyenne (p=6,7377E-07, test significatif si p<0,05) a été obtenue (figure 13), sur un panel de 8 sujets.

**5. Résultats sur l'hydratation**

Dès le 1er soin, une augmentation significative du taux d'hydratation de 56,7% en moyenne (p=2,3195E-12, test significatif si p<0,05) a été obtenue, et dès 3 soins, une augmentation significative du taux d'hydratation de 63,6% en moyenne (p=4,4144E-10, test significatif si p<0,05) a été obtenue (figure 14), sur un panel de 15 sujets.

**Références**

[1] Kozlowska et al, Arch. Dermatol. Res., 290(12) : 661-668, 1998
[2] Yano et al, J. Clin. Invest., 107(4) : 409-417, 2001
[3] Fernandez-Flores A, Saeb-Lima M, Cassarino DS. Histopathology of aging of the hair follicle, J. Cutan. Pathol. 46(7) : 508-519, 2019
[4] Hsieh ST, Lin WM., Modulation of keratinocyte prolifération by skin innervation, J Invest Dermatol. 113(4): 579-586, 1999

**Revendications**

1. Composition cosmétique comprenant de l'éther vanillyl-butylique et un extrait de graines de *Voandzeia subterranea.*

2. Composition cosmétique selon la revendication 1, comprenant en outre un milieu nutricellulaire.

3. Composition cosmétique selon la revendication 2, où le milieu nutricellulaire comprend au moins une vitamine ou composé assimilé à une vitamine, au moins deux acides aminés, et au moins un actif biologique et au moins un sel minéral ;
où lesdits au moins deux acides aminés sont au moins la tyrosine et la phénylalanine.

4. Composition cosmétique selon la revendication 3, où :

- ladite au moins une vitamine ou composé assimilé à une vitamine est choisi dans le groupe constitué de : acide ascorbique (C), biotine (H), pantothénate de calcium (B5), acide folique (B9), Niacine (PP ou B3), Niacinamide, pyridoxal HCl (B6), Riboflavine (B2), acétate de tocophérol (E), thiamine HCl (B1), Ethyl oléate (F Oméga 9), Ethyl linoléate (F Oméga 6), Ethyl linolénate (F Oméga 3) et inositol (B7) ;
- lesdits au moins deux acides aminés sont choisis dans le groupe constitué de : alanine, arginine, acide aspartique, acide glutamique, cystéine, glutamine, glycine, histidine, hydroxyproline, isoleucine, leucine, lysine, méthionine, phénylalanine, proline, sérine, thréonine, tryptophane, tyrosine et valine ;
- ledit au moins un actif biologique est choisi dans le groupe constitué de : Adénosine, Cytosine, Guanine, thymine, adénine, adénosine phosphate (AMP), adénosine triphosphate (ATP), cholestérol, glucose, glutathion, xanthine, uracile, ADN de blé et ARN de levure ;
- ledit au moins un sel minéral est choisi dans le groupe constitué de : Sulfate de Magnésium, Chlorure de Potassium, Nitrate de potassium, Sulfate de Sodium, Chlorure de Calcium, Phosphate de sodium, Chlorure de Sodium et Acétate de Sodium.

5. Composition cosmétique selon l'une quelconque des revendications 2 à 4, comprenant :

- de l'éther vanillyl-butylique ;
- un extrait de graines de *Voandzeia subterranea* ; et
- un milieu nutricellulaire comprenant : acide ascorbique (C), biotine (H), pantothénate de calcium (B5), acide folique (B9), Niacine (PP ou B3), Niacinamide, pyridoxal HCl (B6), Riboflavine (B2), acétate de tocophérol (E), thiamine HCl (B1), Ethyl oléate (F oméga 9), Ethyl linoléate (F Oméga 6), Ethyl linolénate (F Oméga 3), inositol (B7), alanine, arginine, acide aspartique, acide glutamique, cystéine, glutamine, glycine, histidine, hydroxyproline, isoleucine, leucine, lysine, méthionine, phénylalanine, proline, sérine, thréonine, tryptophane, tyrosine, valine, Adénosine, Cytosine, Guanine, thymine, adénine, adénosine phosphate (AMP), adénosine triphosphate (ATP), cholestérol, glucose, glutathion, xanthine, uracile, ADN de blé, ARN de levure, Sulfate de Magnésium, Chlorure de Potassium, Nitrate de potassium, Sulfate de Sodium, Chlorure de Calcium, Phosphate de sodium, Chlorure de Sodium, et Acétate de Sodium.

6. Composition cosmétique selon l'une quelconque des revendications 1 à 5, où ladite composition est un sérum.

7. Composition cosmétique selon l'une quelconque des revendications 1 à 6 destinée à être utilisée pour lutter contre le vieillissement de la peau.

8. Composition cosmétique selon l'une quelconque des revendications 1 à 6 destinée à être utilisée pour limiter la chute des cheveux, et favoriser leur repousse.

9. Composition cosmétique selon l'une quelconque des revendications 1 à 6 destinée à être utilisée pour retarder l'apparition de cheveux blanc.

Fig. 1]

Témoin à J4 : coloration peu intense | Traitement avec composition à J4 : coloration plus intense

[Fig. 2]

Epaisseur d'épiderme à J7

T : Témoin non traité      P

[Fig. 3]

Épaisseur moyenne: 48,6 ± 8,3 μm

**Explant T témoin à J7**

Épaisseur moyenne: 53,7 ± 7,9 μm

**Explant P traité
avec la composition de l'invention A J7**

[Fig. 4]

TF=Très Faible   F=Faible   M =Modéré   AN=Assez Net   N=Net   TN=Très Net   Fo=Fort

EXPLANT T A J7
coloration (verte) fluorescente peu importante

EXPLANT P A J7
coloration (verte) fluorescente plus importante

[Fig. 5]

Evolution of the mean perfusion on one area of the forehead at T0 and 30 minutes after the application of the Sérum Age influx – VR021174.A09

[Fig. 6]

Evolution of the mean perfusion on the scalp at T0 and 30 minutes after the application of the Hair Summum Newlife Concentré VR040140.A09

[Fig. 7]

Evolution of the mean perfusion on the scalp 30 minutes after the application of the HAIR SUMMUM SERUM NEW LIFE VR021195.A05 + DEVICE HAIR SUMMUM

[Fig. 8]

Evolution de la profondeur moyenne des rides
(visioscan ) après 1 soin

-46,4%

T0          T+1 soin

Panel de 8 sujets

| T0 | T+1soin | T+3soins |

Rides profondes          Profondeur des rides fortement diminuée

[Fig.9]

| T0 | T+1soin | T+3 soins |

[Fig. 10]

Evolution de la profondeur moyenne de la ride la plus importante après 1 soin ( projection de franges)

-40,3%

T0

T+1 soin

Panel de 7 sujets

Evolution de la profondeur maximum de la ride la plus importante après 1 soin ( projection de franges)

-40,6%

T0

T+1 soin

Panel de 7 sujets

[Fig. 11]

Evolution de la fermeté après 1 et 3 soins

+77,9%

+51,2%

T0          T+1 soin          T+3 soins

Panel de 12 sujets

[Fig. 12]

Evolution de l'élasticité après 1 et 3 soins

+63,6%

+48,7%

T0          T+1 soin          T+3 soins

Panel de 11 sujets

[Fig. 13]

Evolution de la rugosité après 1 soin

-37,1%

T0

T+1 soin

Panel de 8 sujets

[Fig. 14]

Evolution du taux d'hydratation après 1 et 3 soins

+66,3%

+56,7%

T0

T+1soin

T+3 soins

Panel de 15 sujets

| | Europäisches Patentamt European Patent Office Office européen des brevets | **RAPPORT DE RECHERCHE EUROPEENNE** | Numéro de la demande EP 24 15 4088 |
|---|---|---|---|

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | US 8 445 459 B2 (LABOUREAU JULIEN [FR]; SIMONNET JEAN-THIERRY [FR] ET AL.) 21 mai 2013 (2013-05-21) * colonne 22, ligne 10 - ligne 18 * ----- | 1-9 | INV. A61K8/34 A61K8/97 A61Q7/00 A61Q19/08 |
| Y | US 2019/269626 A1 (WU PEI-HSUAN [TW] ET AL) 5 septembre 2019 (2019-09-05) * revendications 1,2 * ----- | 1-9 | |
| Y | US 6 391 320 B1 (PAULY GILLES [FR]) 21 mai 2002 (2002-05-21) * le document en entier * ----- | 1-9 | |
| Y | DATABASE GNPD [Online] MINTEL; 21 septembre 2021 (2021-09-21), anonymous: "Fit Body Gel Cream", XP055941641, Database accession no. 8864841 * le document en entier * ----- | 1-9 | |
| Y | "SENSATES AND THEIR APPLICATION IN CONSUMER PRODUCTS", RESEARCH DISCLOSURE, KENNETH MASON PUBLICATIONS, HAMPSHIRE, UK, GB , vol. 691, no. 54 1 novembre 2021 (2021-11-01), XP007149765, ISSN: 0374-4353 Extrait de l'Internet: URL:https://www.researchdisclosure.com/database/RD691054 [extrait le 2021-10-11] * le document en entier * ----- -/-- | 1-9 | DOMAINES TECHNIQUES RECHERCHES (IPC) A61K A61Q |

Le présent rapport a été établi pour toutes les revendications

| Lieu de la recherche | Date d'achèvement de la recherche | Examinateur |
|---|---|---|
| Munich | 14 mai 2024 | Yon, Jean-Michel |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Europäisches
Patentamt

European
Patent Office

Office européen
des brevets

## RAPPORT DE RECHERCHE EUROPEENNE

**Numéro de la demande**

EP 24 15 4088

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | DATABASE GNPD [Online] MINTEL; 20 mars 2023 (2023-03-20), anonymous: "Cream Mask Refining", XP093091325, Database accession no. 10619748 * le document en entier * ----- | 1-9 | |
| Y | DATABASE GNPD [Online] MINTEL; 3 février 2022 (2022-02-03), anonymous: "Awaken Peptide Eye Gel", XP093091329, Database accession no. 9363302 * le document en entier * ----- | 1-9 | |

**DOMAINES TECHNIQUES RECHERCHES (IPC)**

Le présent rapport a été établi pour toutes les revendications

| Lieu de la recherche | Date d'achèvement de la recherche | Examinateur |
|---|---|---|
| Munich | 14 mai 2024 | Yon, Jean-Michel |

CATEGORIE DES DOCUMENTS CITES

X : particulièrement pertinent à lui seul
Y : particulièrement pertinent en combinaison avec un
  autre document de la même catégorie
A : arrière-plan technologique
O : divulgation non-écrite
P : document intercalaire

T : théorie ou principe à la base de l'invention
E : document de brevet antérieur, mais publié à la
  date de dépôt ou après cette date
D : cité dans la demande
L : cité pour d'autres raisons
................................................................
& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 24 15 4088

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

14-05-2024

| Document brevet cité au rapport de recherche | | Date de publication | Membre(s) de la famille de brevet(s) | | Date de publication |
|---|---|---|---|---|---|
| US 8445459 | B2 | 21-05-2013 | BR | PI0913763 A2 | 28-02-2012 |
| | | | CN | 101766546 A | 07-07-2010 |
| | | | EP | 2206491 A1 | 14-07-2010 |
| | | | ES | 2598499 T3 | 27-01-2017 |
| | | | FR | 2940610 A1 | 02-07-2010 |
| | | | JP | 2010155836 A | 15-07-2010 |
| | | | KR | 20100080439 A | 08-07-2010 |
| | | | US | 2010168055 A1 | 01-07-2010 |
| US 2019269626 | A1 | 05-09-2019 | US | 2019269626 A1 | 05-09-2019 |
| | | | WO | 2019166020 A1 | 06-09-2019 |
| US 6391320 | B1 | 21-05-2002 | AT | E211904 T1 | 15-02-2002 |
| | | | AU | 6405298 A | 20-10-1998 |
| | | | CA | 2284667 A1 | 01-10-1998 |
| | | | DE | 69803169 T2 | 22-08-2002 |
| | | | EP | 0973495 A1 | 26-01-2000 |
| | | | ES | 2172117 T3 | 16-09-2002 |
| | | | FR | 2761264 A1 | 02-10-1998 |
| | | | JP | 2001518924 A | 16-10-2001 |
| | | | KR | 20010005588 A | 15-01-2001 |
| | | | US | 6391320 B1 | 21-05-2002 |
| | | | WO | 9842305 A1 | 01-10-1998 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2202529

**Littérature non-brevet citée dans la description**

- **KOZLOWSKA et al.** *Arch. Dermatol. Res,* 1998, vol. 290 (12), 661-668
- **YANO et al.** *J. Clin. Invest.,* 2001, vol. 107 (4), 409-417
- **FERNANDEZ-FLORES A ; SAEB-LIMA M ; CASSARINO DS.** Histopathology of aging of the hair follicle. *J. Cutan. Pathol.,* 2019, vol. 46 (7), 508-519
- **HSIEH ST ; LIN WM.** Modulation of keratinocyte prolifération by skin innervation. *J Invest Dermatol.,* 1999, vol. 113 (4), 579-586